# EUROPÄISCHE PATENTSCHRIFT

(11) **EP 1 276 853 B1**
(45) Veröffentlichungstag und Bekanntmachung des Hinweises auf die Patenterteilung: **16.08.2006**
(21) Anmeldenummer: 01929607.8
(22) Anmeldetag: 26.04.2001
(51) Int. Cl.: C12N 7/01, A61K 38/00, A61P 37/02, C07K 14/135, A61K 38/16

(54) **PNEUMOVIRUS NS PROTEINE ANTAGONISIEREN DIE INTERFERON (IFN) ANTWORT**
PNEUMOVIRUS NS PROTEINS ANTAGONISING THE INTERFERON (IFN) RESPONSE
PROTEINES NS DE PNEUMOVIRUS ANTAGONISANT LA REPONSE D'INTERFERON (IFN)

(30) Priorität: 26.04.2000 DE 10020505
(43) Veröffentlichungstag der Anmeldung: 22.01.2003
(73) Patentinhaber: Wyeth, Madison, New Jersey 07940 (US)
(72) Erfinder: Conzelmann, Karl-Klaus, Prof.Dr., 82061 Neuried (DE)
(74) Vertreter: Grund, Martin
(86) Internationale Anmeldenummer: PCT/EP2001/004740
(87) Internationale Veröffentlichungsnummer: WO 2001/081554

(56) Entgegenhaltungen:
- WO-A-98/02530
- WO-A-99/64068
- BUCHHOLZ U J ET AL: "CHIMERIC BOVINE RESPIRATORY SYNCYTIAL VIRUS WITH GLYCOPROTEIN GENE SUBSTITUTIONS FROM HUMAN RESPIRATORY SYNCYTIAL VIRUS (HRSV): EFFECTS ON HOST RANGE AND EVALUATION AS A LIVE-ATTENUATED HRSV VACCINE" JOURNAL OF VIROLOGY, THE AMERICAN SOCIETY FOR MICROBIOLOGY, US, Bd. 74, Nr. 3, Februar 2000 (2000-02), Seiten 1187-1199, XP000972255 ISSN: 0022-538X in der Anmeldung erwähnt
- BUCHHOLZ U J ET AL: "GENERATION OF BOVINE RESPIRATORY SYNCYTIAL VIRUS (BRSV) FROM CDNA: BRSV NS2 IS NOT ESSENTIAL FOR VIRUS REPLICATION IN TISSUE CULTURE, AND THE HUMAN RSV LEADER REGION ACTS AS A FUNCTIONAL BRSV GENOME PROMOTER" JOURNAL OF VIROLOGY, THE AMERICAN SOCIETY FOR MICROBIOLOGY, US, Bd. 73, Nr. 1, Januar 1999 (1999-01), Seiten 251-259, XP000972290 ISSN: 0022-538X in der Anmeldung erwähnt
- SCHLENDER J ET AL: "BOVINE RESPIRATORY SYNCYTIAL VIRUS NONSTRUCTURAL PROTEINS NS1 AND NS2 COOPERATIVELY ANTAGONIZE ALPHA/BETA INTERFERON-INDUCED ANTIVIRAL RESPONSE" JOURNAL OF VIROLOGY, THE AMERICAN SOCIETY FOR MICROBIOLOGY, US, Bd. 74, Nr. 18, September 2000 (2000-09), Seiten 8234-8242, XP000972437 ISSN: 0022-538X

## Beschreibung

### HINTERGRUND DER ERFINDUNG

### Technisches Gebiet der Erfindung

Die vorliegende Erfindung bezieht sich auf die Verwendung eines Pneumovirus NS2 Proteins oder einer NS2 kodierende Nukleinsäuresequenz für die Herstellung einer pharmazeutischen Formulierung zur Reduzierung der durch Interferon (IFN) vermittelten Immunantwort.

Die vorliegende Erfindung bezieht sich außerdem auf die Verwendung eines Pneumovirus NS1 Proteins und NS2 Proteins oder einer Nukleinsäure, die Pneumovirus NS1 Protein und NS2 Protein kodiert, für die Herstellung einer pharmazeutischen Formulierung zur Reduzierung der durch Interferon (IFN) vermittelten Immunantwort. Die Erfindung bezieht sich außerdem auf rekombinante Pneumoviren, insbesondere Respiratorische Syncytialviren (RSV) mit erhöhter, verminderter, oder fehlender Resistenz gegenüber der durch Interferon (IFN) vermittelten Immunantwort des Wirts, rekombinante Viren mit erhöhter Resistenz gegen die durch Interferon (IFN) vermittelte Immunantwort und die Verwendung dieser Viren in pharmazeutischen Formulierungen, z.B. als Impfstoffe.

### Beschreibung des Stands der Technik

Das bovine Respiratorische Syncytialvirus (BRSV) ist das bedeutendste ätiologische Agens von Krankheiten des Respirationstrakts bei Kälbern und verursacht wesentliche wirtschaftliche Verluste (40;45). Die Immunantwort und die Pathologie in Kälbern gleicht den Symptomen, die durch das humane Respiratorische Syncytialvirus (HRSV) verursacht wird, das immer noch die Hauptursache schwerer Bronchiolitiden und Pneumonien bei Säuglingen und Kleinkindern in der ganzen Welt darstellt (9). Die molekulare Klonierung von HRSV und BRSV sowie von einem verwandten Virus, dem Pneumovirus der Maus (Pneumonia Virus of Mice; PVM) hat nicht nur eine sehr nahe Verwandtschaft zwischen BRSV und HRSV bestätigt, sondern hat auch wesentliche gemeinsame Unterschiede zu anderen Mitgliedern der Paramyxoviridae Familie aufgedeckt, was zur Etablierung der Subfamilie Pneumovirinae innerhalb der Paramyxoviridae Familie gerührt hat (36;37). Die RSV Viren und das PVM bilden innerhalb der Subfamilie den Genus Pneumovirus, das Avian Pneumovirus (APV) als bisher einziger Vertreter den Genus Metapneumovirus.

Wie bei allen Mitgliedern der Ordnung Mononegavirales, ist die circa 15 kb große Genom-RNA von RSV und PVM in einem Ribonukleoprotein (RNP) Komplex enthalten, der als Matrize für die sequentielle Transkription von Genen fungiert (25:49). Elf Proteine werden von 10 Transkriptionseinheiten exprimiert, die in der Reihenfolge 3'-NS1-NS2-N-P-M-SH-G-F-M2-L-5' angeordnet sind (5;9;30;31). Die kodierten Proteine umfassen fünf RNP-assoziierte Proteine: das Nukleoprotein (N), das Phosphoprotein (P), die große katalytische Untereinheit der RNA Polymerase (L), und einen Transkriptions-Elongations-Faktor (M2-1), der durch das erste von zwei überlappenden Offenen Leserastern des M2 Gens kodiert wird (8;17;27;38). Das zweite Offene Leseraster der M2 Transkriptionseinheit (M2-2) kodiert, wie beschrieben wurde, ein nicht essentielles Protein (1), das vermutlich an der Regulierung der RNA Synthese beteiligt ist (4;28). Drei Virusproteine sind mit der Virushülle assoziiert: das Fusionsprotein F, das vermeintliche Attachment Protein G, und ein kleines hydrophobes Protein SH.

Das Vorkommen von zwei Nichtstrukturprotein Genen an der 3'-terminalen Position des Genoms unterscheidet die Mitglieder des Pneumovirus-Genus von allen anderen Vertretern der Ordnung Mononegavirales. Aufgrund der 3'-nahen Lage werden die NS Gene übermäßig transkribiert. Die kodierten Proteine wurden in infizierten Zellen nachgewiesen (10;16). Die BRSV NS Gene kodieren Polypeptide mit 136 und 124 Aminosäuren. Der Vergleich mit den NS Proteinen von HRSV aus den Untergruppen A und B zeigte Aminosäure-Homologien von 69% und 68% für die NS1 Proteine und von 84% und 83% für die NS2 Proteine (5;34). Die NS1 und NS2 Proteine des PVM zeigen keine signifikante Homologie zu den RSV NS Genen (ca. 20 % Identität), besitzen jedoch analoge Funktionen. Die abgeleiteten Sequenzen erbrachten jedoch keine offensichtlichen Hinweise auf die Funktion von NS Proteinen im Lebenszyklus des Virus. Wie berichtet wurde, ist das HRSV NS1 Protein mit dem M Protein assoziiert, während das NS2 Protein keine nachweisbare Assoziierung mit RSV Strukturproteinen zeigte, was auf verschiedene Funktionen von NS1 und NS2 hinweist (16;47). Eine hemmende Funktion von NS1 bei der Virus RNA Transkription wurde kürzlich durch Experimente, in denen künstliche HRSV Minigenome in Abwesenheit oder Anwesenheit von NS1 angezogen wurden, nahegelegt. In derselben Studie wurde auch für das NS2 ein hemmender, aber weitaus weniger deutlich ausgeprägter Effekt für das NS2 beobachtet (3).

Vor kurzem etablierte Verfahren zur Herstellung ("Recovery") von infektiösen Negativ Strang RNA-Viren von cDNA (11) ermöglichten die Erzeugung von rekombinanten humanen (8;29) und bovinen RSV (5) und die Untersuchung von individuellen Proteinfunktionen im Virus Kontext. Die erfolgreiche Herstellung von lebensfähigen NS2 Gen-Deletionsmutanten bestätigte, daß NS2. nicht essentiell für die Virusreplikation in Zellkultur ist (5;43). Das Muster und die relativen Mengen an mRNAs und Gesamt-Länge RNA, die in infizierten Zellen produziert wurden, waren nicht deutlich verändert. Die Deletionsmutanten waren im Vergleich zum Ausgangsvirus jedoch attenuiert, zeigten langsamere Wachstumsraten und erbrachten niedrigere infektiöse Virustiter. Obwohl das NS2 nicht essentiell ist, stellt es somit einen akzessorischen Faktor dar, der in der Lage ist, das Viruswachstum durch einen bisher unbekannten Mechanismus wesentlich zu unterstützen. Die Funktion der NS Proteine bleibt jedoch bisher unbekannt.

WO 99/64068 bezieht sich auf attenuierte Negativ-Strang RNA Viren mit einer veränderten Interferon Antagonisten Aktivität für die Verwendung als Vakzine und pharmazeutischen Formulierungen. WO 98/02530 bezieht sich auf die Produktion attenuierter RSV Vakzine, die aus klonierten Nukleotidsequenzen gewonnen werden. Buchholz et al. (Buchholz et al., Journal of Virology, Jan 1999, Seiten 251-259) bezieht sich auf die Erzeugung VOn BRSV aus cDNA.

### ZUSAMMENFASSUNG DER ERFINDUNG

Diese Erfindung beruht auf der Entdeckung, daß die NS 1 und/oder NS2 Proteine von RSV und RSV-ähnlichen Viren, insbesondere aus der Gattung Pneumovirus (RSV und PVM), und die im weiteren alle als "RSV" bezeichnet werden, antagonistischen Effekt auf die IFN vermittelte Immunantwort besitzen.

Die vorliegende Erfindung bezieht sich auf die Verwendung eines Pneumovirus NS2 Proteins oder einer NS2 kodierende Nukleinsäuresequenz für die Herstellung einer pharmazeutischen Formulierung zur Reduzierung der durch Interferon (IFN) vermittelten Immunantwort.

Die vorliegende Erfindung bezieht sich außerdem auf die Anwendung eines RSV NS1 und NS2 Proteins oder einer RSV NS1-kodierenden Nukleinsäuresequenz, und einer RSV NS2-kodierenden Nukleinsäuresequenz zur Herstellung einer pharmazeutischen

Formulierung zur Reduzierung der durch IFN vermittelten Immunantwort, vorzugsweise durch IFN alpha und/oder beta.

In einer bevorzugten Ausführungsform der vorliegenden Erfindung wird das RSV NS1 Protein in Kombination mit dem NS2 Protein zur Herstellung einer pharmazeutischen Formulierung benutzt, oder eine RSV NS1 Protein-kodierende Nukleinsäuresequenz wird in Kombination mit einer RSV NS2-Protein-kodierenden Nukleinsäuresequenz zur Herstellung einer pharmazeutischen Formulierung benutzt, um die IFN-vermittelte Immunantwort zu reduzieren. "In Kombination" bezieht sich entsprechend der vorliegenden Erfindung auf eine Verabreichung, die entweder gleichzeitig oder aufeinanderfolgend geschieht.

In einem weiteren Aspekt der vorliegenden Erfindung wird RSV NS2 Protein oder die RSV NS1 und NS2 Proteine benutzt, um ein nicht verwandtes Virus vor der EFN-vermittelten Immunantwort zu schützen. Vorzugsweise handelt es sich bei den RSV NS1 und/oder NS2 Proteinen um die BRSV NS Proteine. Die NS1 and NS2 Proteine schützen auf kooperative Weise ein nicht verwandtes Virus. "Nicht verwandtes Virus" gemäß der vorliegenden Erfindung bezieht sich auf Viren, die RSV NS 1 und/oder NS2 Protein nicht exprimieren. In einer bevorzugten Ausführungsform ist das Virus das Tollwutvirus. Die RSV NS 1 und/oder NS2 Proteine werden vorzugsweise von der viralen Nukleinsäure exprimiert.

In einem weiteren Aspekt umfassen die ober genammten pharmazeutische Formulierungen der Erfindung, weiterhin einen Impfstoff. Bevorzugterweise resultiert eine Verabreichung des linpfstoffes zusammen mit den pharmazeutischen Formulierungen in einer verlangsamten Eliminierung des Impfstoffes aus dem Körper, dem er verabreicht wird.

Die Erfindung bezieht sich auch auf die Anwendung von veränderten RSV NS2 Proteinen für die Herstellung einer pharmazeutischen Formulierung zur Modulierung der IFN-vermittelten Immunantwort. In einer bevorzugten Ausführungsform resultiert die Veränderung in einer Verstärkung der IFNmodulierenden Aktivität des NS2 Proteins.

Die vorliegende Erfindung bezieht sich auch auf die Verwendung eines rekombinanten RSV, das eine veränderte NS2-kodierende Nukleinsäuresequenz trägt, zur Herstellung eines Impfstoffes, wobei die Veränderung die Fähigkeit des Virus, einer IFN-vermittelten Immunantwort zu entgehen, vermindert oder zerstört wird. In einer bevorzugten Ausfühzwagsform ist die veränderte NS2-kodierende Nukleinsäuresequenz homolog oder heterolog zu dem besagten rekombinanten RSV. Besonders bevorzugt werden Anwendungen, in denen das rekombinante RSV von humanem, bovinen RSV oder von PVM abgeleitet ist.

### DETAILLIERTE BESCHREIBUNG DER ERFINDUNG

Um die Funktion der RSV Proteine genauer zu untersuchen, wurden BRSV Deletionsmutanten, denen das NS1 Gen oder die NS1 und NS2 Gene fehlen, erzeugt und deren Verhalten in unterschiedlichen Zellinien untersucht.

Der erste Hinweis auf eine erhöhte Sensitivität der NS Deletionsmutanten gegenüber Wirtszell-Faktoren wurde nach Infektion von MDKB-Zellen beobachtet, die voll empfänglich für eine wt BRSV Infektion sind, und die höhere wt BRSV Titer als alle anderen getesteten Zellinien erbringen. Im Gegensatz dazu wuchsen Viren, denen ein oder beide NS Gene fehlen, am schlechtesten in MDKB-Zellen, während in anderen Zellinien wie Vero oder BSR das Fehlen der NS Gene lediglich eine 10-fache Reduzierung der infektiösen Titer verursachte. Durch Kokultivierungs-Experimente wurden Typ I Interferone als die von BRSV-infizierten MDKB-Zellen produzierten ausschlaggebenden Wirtszellfaktoren identifiziert. Offensichtlich wird in infizierten MDBK Kulturen durch autokrine und parakrine Stimulation von Zellen ein antiviraler Status induziert. Während wt BRSV in der Lage ist, diese Antwort entgegenzuwirken, ist keine der NS Deletionsmutanten dazu in der Lage. Im Gegensatz dazu fehlen den Vero-Zellen die Typ I Interferon Gene (15;46), so daß die Virus-Infektion nicht in der Induktion eines antiviralen Status resultiert und den NS Deletionsmutanten das Wachstum ermöglicht wird. Obwohl Vero-Zellen nicht in der Lage sind, Interferon zu produzieren, können sie durch JAK/STAT vermittelte Signalübertragung über den IFN alpha Rezeptor (IFNAR) Komplex auf Stimulation durch exogenes Interferon reagieren. Bovine Interferone, die von den infizierten MDKB-Zellen sekretiert werden, induzierten in Vero "Responder" Zellen eine antivirale Antwort, welche das Wachstum der NS Deletionsmutanten, nicht aber das des wt BRSV unterdrückten. Der durch die MDBK Überstände verursachte antivirale Effekt wurde durch Inkubation von Vero-Zellen mit einem Antikörper, der die Bindung von IFN an den IFNAR blockiert, verhindert. Die einzig aktiven Bestandteile der MDBK Zell-Überstände bei der Induktion der antiviralen Antwort in Vero-Zellen waren somit IFN alpha und/oder IFN beta.

Der hemmende Effekt auf die NS Deletionsmutanten in Vero-Zellen, die mit Überständen von MDKB-Zellen behandelt wurden, war mit einer maximalen 7-fachen Reduktion der Doppel-Deletionsmutante schwach, und somit nicht vergleichbar mit der schweren Hemmung der NS Deletionsmutanten in MDKB-Zellen. Dies kann verschiedenen Faktoren zugeschrieben werden. Die Stimulierung der von Primaten stammenden Vero-Zellen mit den heterologen IFN bovinen Ursprungs ist vermutlich weniger effizient als die Stimulierung von MDBK. Ähnlich der Situation beim Menschen, wurden verschiedene bovine IFN alpha (Typ 2 bis 8) und IFN beta (Typ 1 und 3), die unterschiedliche biologische Aktivität zeigen, identifiziert (7). Außerdem scheinen die antiviralen Mechanismen von Vero-Zellen weniger wirkungsvoll zu sein als die von MDBK. Überstände von aktivierten und infizierten Makrophagen, die dafür bekannt sind, größere Mengen an Typ I Interferon zu sekretieren als andere Zellen, bewirkten eine verstärkte, bis zu 50-fache, Reduzierung der NS Deletionsmutanten in Vero "Responder" Zellen. Durch Stimulierung von Vero-Zellen mit rekombinantem humanem IFN alpha A/D oder IFN beta konnte die Replikation der NS Deletionsmutanten dosisabhängig gehemmt werden, wobei 500 Einheiten die Replikationsaktivität nahezu vollständig hemmte.

Somit werden die RSV Proteine durch die vorliegende Erfindung als Antagonisten der IFN-vermittelten Wirtszell Antwort identifiziert.

Darüber hinaus konnte durch Verwendung eines anderen Negativ-Strang RNA Virus, des Tollwutvirus, als Vektor für die Expression der BRSV-abgeleiteten NS Gene gezeigt werden, daß die Aktivität der beiden NS Proteine NS1 und NS2 die IFN Resistenz eines nicht verwandten Virus erhöhen kann. Ein weiterer Befund war, daß die NS1 und NS2 Proteine von verschiedenen RSV stammen können.

Insgesamt konnte den RSV NS Proteinen eine wichtige biologische Funktion zugeordnet werden, sie vermitteln den Schutz des Virus vor der zellulären, durch Interferon vermittelten antiviralen Mechanismen (IFN antagonistische Wirkung). Darüber hinaus wurde unerwarteterweise entdeckt, daß durch die Anwendung von beiden NS Proteinen zusammen die betreffende IFN antagonistische Wirkung potenziert wurde. Dies ist das erste Beispiel für eine Kooperation von zwei Virusproteinen zur Antagonisierung von Interferon.

Die Anpassung viraler Proteine daran, in Zellen ihres natürlichen Wirtes angeborenen Antworten entgegenzuwirken, ist wahrscheinlich ein wichtiger Faktor bei der Bestimmung des viralen Wirtsspektrums und kann Viren daran hindern, Speziesbarrieren zu überwinden (13;23;26). Das V Protein des Simian Virus 5 (SV5) ist zum Beispiel in der Lage, die Aktivierung der Interferon-responsiven Gene in Primatenzellen zu blockieren, nicht jedoch in murinen Zellen (14). Dies könnte der maßgebliche Mechanismus sein, der eine produktive SV5-Infektion von Mäusen, sogar von SCID-Mäusen, verhindert (13). HRSV, das humane Pendant zu BRSV, wurde kürzlich als resistent gegenüber IFN induzierter antiviraler Aktivität in humanen Zellen beschrieben (2) und wir schließen aus unseren Befunden, dass die HRSV NS Proteine für die Antagonisierung der IFN Antwort in humanen Zellen optimiert sind, und besser als in Zellen anderen Ursprungs.

Tatsächlich kann der Beitrag der NS Proteine zur Empfänglichkeit von Wirten für eine RSV Infektion erklären, weshalb die nahe verwandten Viren ein stark eingeschränktes Wirtsspektrum besitzen. BRSV und HRSV sind in der Lage, in humane, bovine und murine Zellen einzudringen; die unterschiedliche Fähigkeit der NS Proteine, die wirtsspezifischen Abwehrmechanismen zu antagonisieren könnte dann darüber entscheiden, ob das Virus eliminiert wird oder nicht. Dies wird auch durch frühere Befunde unterstützt. Das Wachstum von HRSV in primären Maus Embryo (ME) Zellen war deutlich eingeschränkt, nach Zugabe von anti Maus IFN Serum zum Medium war jedoch die Virusausbeute erhöht und die Infektion breitete sich im gesamten Zellrasen aus (26). Außerdem waren rekombinante BRSV, in denen, um das Eindringen in Primatenzellen zu erleichtern, die G und F Oberflächenproteine gegen die des HRSV ausgetauscht waren, wenig kompetenter bei der Replikation in Schimpansen als BRSV. Die Infektion blieb jedoch stark eingeschränkt und war für die Induktion eines Schutzes gegen eine homologe HRSV-Belastungsinfektion nicht ausreichend (6). Aus unseren Beobachtungen schließen wir, dass die geringe Wirksamkeit der BRSV NS Proteine bei der Antagonisierung der Abwehrmechanismen von Primaten die hauptsächliche Determinante des Wirtsspektrums darstellt.

Für das Design von wirksamen, attenuierten RSV Lebendvakzinen haben unsere Befunde deshalb wichtige Implikationen. Die Deletion von NS1 oder NS2, oder von beiden Genen resultiert in über-attenuierten Viren, die nicht in der Lage sind, einer jeglichen Interferon Antwort zu entkommen. Überdies können Impfstoffe entworfen werden, die durch den reziproken Austausch der NS Proteine zwischen verschiedenen RSV, zum Beispiel BRSV und HRSV, und insbesondere, um BRSV und HRSV Vakzinen zu erzeugen, die intermediäre Fähigkeit besitzen, den angeborenen bovinen und humanen Abwehrmechanismen zu entgehen. Zusätzlich können Mutationen in den NS Proteinen angefertigt werden, die die IFN antagonistische Aktivität nur partiell zerstören.

Das NS2 Protein oder eine NS2 kodierende Nukleinsäuresequenz gemäß dieser Erfindung ist geeignet zur Herstellung einer pharmazeutischen Formulierung zur Abschwächung der durch Interferon vermittelten Immunantwort.

Darüber hinaus sind RSV NS1 und NS2 Proteine oder NS 1-kodierende und NS2-kodierende Nukleinsäuresequenzen gemäß dieser Erfindung auch geeignet zur Herstellung von pharmazeutischen Formulierungen zur Abschwächung der durch IFN vermittelten Immunantwort. In einer bevorzugten Ausführungsform wird das RSV NS1 in Kombination mit dem NS2 Protein zur Herstellung einer pharmazeutischen Formulierung eingesetzt. Die pharmazeutische Formulierung beinhaltet eine wirksame Menge an NS1 und NS2, um die durch IFN vermittelte Immunantwort in einem Tier oder Menschen zu verändern, und einen pharmazeutisch vertretbaren Träger oder Verdünner. Die pharmazeutische Formulierung kann mit konventionellen Techniken hergestellt werden. So kann zum Beispiel die pharmazeutische Formulierung der vorliegenden Erfindung durch Zumischung einer gewünschten Menge eines RSV NS1 und/oder NS2 Proteins oder einer NS1-kodierenden Nukleinsäuresequenz und/oder einer NS2-kodierenden Nultleinsäuresequenz zu steriler isotonischer Lösung, die durch einen angemessenen Puffer im pH Wert an einen pH von ungefähr 6.0 angepaßt ist, hergestellt werden.

Die pharmazeutische Formulierung gemäß der vorliegenden Erfindung kann konventionelle Bestandteile enthalten, z.B. einen pharmazeutisch akzeptablen Träger oder Verdünnungsmittel wie Saline, pH-Regulator, Puffer, Konservierungsmittel und ähnliches. Derartige Bestandteile sind dem Fachmann bekannt und können von ihm ausgewählt werden.

Der Ausdruck "IFN" gemäß der vorliegenden Erfindung bezieht sich vorzugsweise auf Typ I Interferon, nämlich Interferon α and β.

Der Ausdruck "IFN vermittelte Immunantwort" bezieht sich hierin auf die durch die Wirkung von Interferon, insbesondere Interferon α und β, induzierten immunisierenden und/oder antiviralen Effekte als Antwort auf z.B. eine Virusinfektion, wie z.B. erhöhte Expression von MHC-Glykoproteinen, Aktivierung von antiviralen Mechanismen wie die Zerstörung von Virus-infizierten Zellen, oder die Hemmung der Virusreplikation.

"Reduzierung der IFN-vermittelten Immunantwort" bedeutet, dass die immunisierenden und/oder antiviralen Effekte abgeschwächt werden, die durch die Wirkung von Interferon, insbesonders Interferon α und β induziert werden, z.B. als eine Antwort auf eine Virus Infektion, wie z.B. erhöhte Expression von MHC-Glykoproteinen, Aktivierung von antiviralen Mechanismen wie die Zerstörung von Virus-infizierten Zellen, oder die Hemmung der Virusreplikation.

"IFN antagonistische Aktivität" gemäß der vorliegenden Erfindung bedeutet, dass die durch die Wirkung von Interferon, insbesondere Interferon α und β, induzierten immunisierenden und/oder antiviralen Effekte als Antwort auf z.B. eine Virus Infektion, wie z.B. erhöhte Expression von MHC-Glykoproteinen, Aktivierung von antiviralen Mechanismen wie die Zerstörung von Virus-infizierten Zellen, oder die Hemmung der Virusreplikation abgeschwächt oder ausgeschaltet werden.

Die Ausdrücke "modifiziert" oder "verändert" gemäß der vorliegenden Erfindung bezieht sich auf den Wildtyp, der als Vergleichsmaßstab dient.

"Nukleinsäuresequenz" wie hier gebraucht, bedeutet jegliche fortlaufende Sequenz von Nukleotidbasen und kann aus Ribonukleinsäure und Desoxyribonukleinsäure bestehen. Vorzugsweise ist die Nukleinsäuresequenz cDNA.

"NS1 und/oder NS2-kodierende Nukleinsäuresequenz homolog oder heterolog zu einem RSV" bedeutet, dass die NS1 und/oder NS2-kodierende Nukleinsäuresequenz von der gleichen oder einer anderen RSV Spezies oder von einem anderen Virus aus der Gattung der Pneumoviren, z.B. PVM, abgeleitet ist.

Veränderungen in einer Protein Sequenz des RSV NS1 oder RSV NS2 schließen einzelne oder mehrfache Aminosäureaustausche, Deletionen und Insertionen ein. Vorzugsweise beeinflussen die Modifikationen nicht die biologische Aktivität der Proteine bei der Reduzierung der IFN-vermittelten Immunantwort oder der Antagonisierung von IFN. Aminosäure-Insertionsvarianten gemäß der vorliegenden Erfindung schließen amino- und/oder carboxyterminale Fusionen und Intra-Sequenz Insertionen von einzelnen oder mehreren Aminosäuren ein. Aminosäure Insertionsvarianten sind solche, bei denen eine oder mehrere Aminosäuren in eine bestimmte Stelle im Protein eingeführt werden; zufallsmäßige Insertion ist jedoch ebenfalls möglich, in Verbindung mit einem geeigneten Screening des resultierenden Produktes. Deletionsvarianten sind durch die Entfernung einer oder mehrerer Aminosäuren aus der Sequenz gekennzeichnet. Substitutions-Aminosäurevarianten sind solche, in denen mindestens ein Rest der Sequenz entfernt und durch einen anderen Rest an dessen Stelle ersetzt ist. Vorzugsweise ist die Sequenz des veränderten NS 1 oder NS 2 Proteins mindestens 40%, bevorzugterweise mindestens 50%, noch bevorzugter mindestens 80% oder mindestens 90%, insbesondere 95 % homolog zum Wildtyp.

Bevorzugte Veränderungen sind an den Positionen, die zwischen den Spezies nicht konserviert sind. Vorzugsweise wird in einer solcher Veränderung eine Aminosäure durch eine von ähnlicher Größe und Polarität ersetzt. Bezüglich der Art der durchführbaren Austausche können zunächst Analysen der Häufigkeit von Aminosäurenaustauschen zwischen homologen Proteinen bei unterschiedlichen Organismen herangezogen werden. Auf der Basis solcher Analysen sind konservative Austausche als Austausche innerhalb der im folgenden angeführten Gruppen definiert:
1. kleine aliphatische, nichtpolare oder schwach polare: Ala, Ser, Thr (Pro, Gly)
2. negativ geladene und ihre Amide: Asn, Asp, Glu, Gln
3. positiv geladene: His, Arg, Lys
4. große aliphatische, nichtpolare: Met, Leu, Ile, Val (Cys)
5. große aromatische: Phe, Tyr, Trp.

Drei Aminosäuren sind aufgrund ihrer speziellen Rolle bei der Proteinarchitektur in Klammern gesetzt. Gly ist die einzige Aminosäure ohne Seitenkette und verleiht deshalb der Peptidkette Flexibilität. Pro besitzt eine ungewöhnliche Geometrie, was die Flexibilität der Kette stark beschränkt. Cys kann an Disulfid-Brücken beteiligt sein.

Außerdem können Veränderungen, wie oben ausgeführt, in die Proteinsequenz des RSV NS 1 oder RSV NS2 eingeführt werden, die in einer Verstärkung oder Abschwächung der biologischen Aktivität der Proteine resultiert, um die IFN vermittelte Immunantwort abzuschwächen oder IFN zu antagonisieren.

### KURZE BESCHREIBUNG DER ZEICHNUNGEN

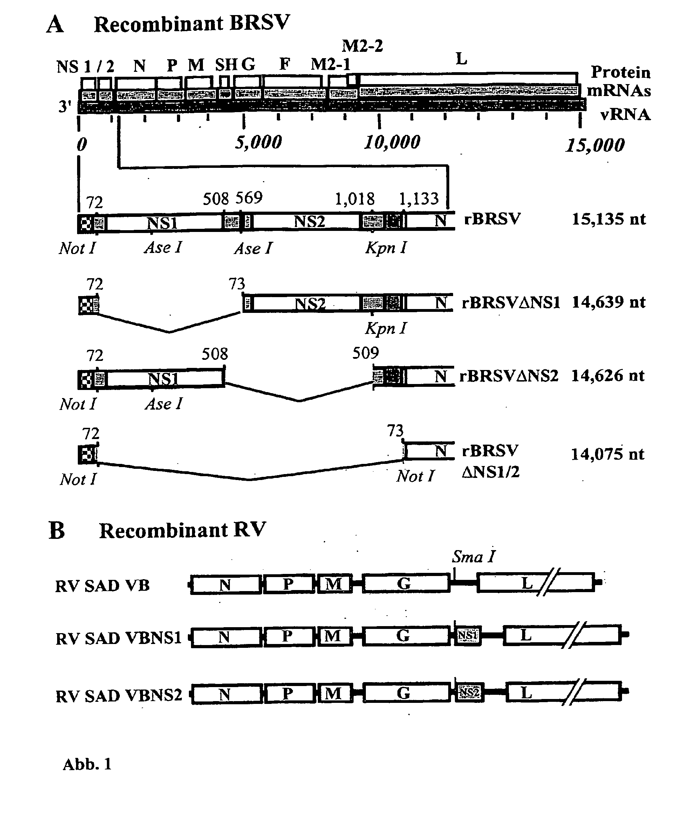

**Abb. 1.** (A) Diagramm der Genome von rekombinanten BRSV. Die Lage der Transkripte (graue Balken) und Protein-kodierenden Raster (weiße Balken) sind relativ zum viralen Genom (vRNA) (schwarze Balken) gezeigt. In der Vergrößerung wird die Organisation des Gesamtlänge-Virus mit den NS Deletionsmutanten verglichen. Die Leader-RNA ist durch horizontale Streifen bezeichnet; die relative Positionen entsprechender Nukleotide und für die Klonierung benutzter Restriktionsstellen sind angegeben. (B) Organisation der rekombinanten Tollwutviren (RV), die genetisch markierte BRSV NS1 oder BRSV NS2 ORFs zwischen den RV G und L Genen besitzen.

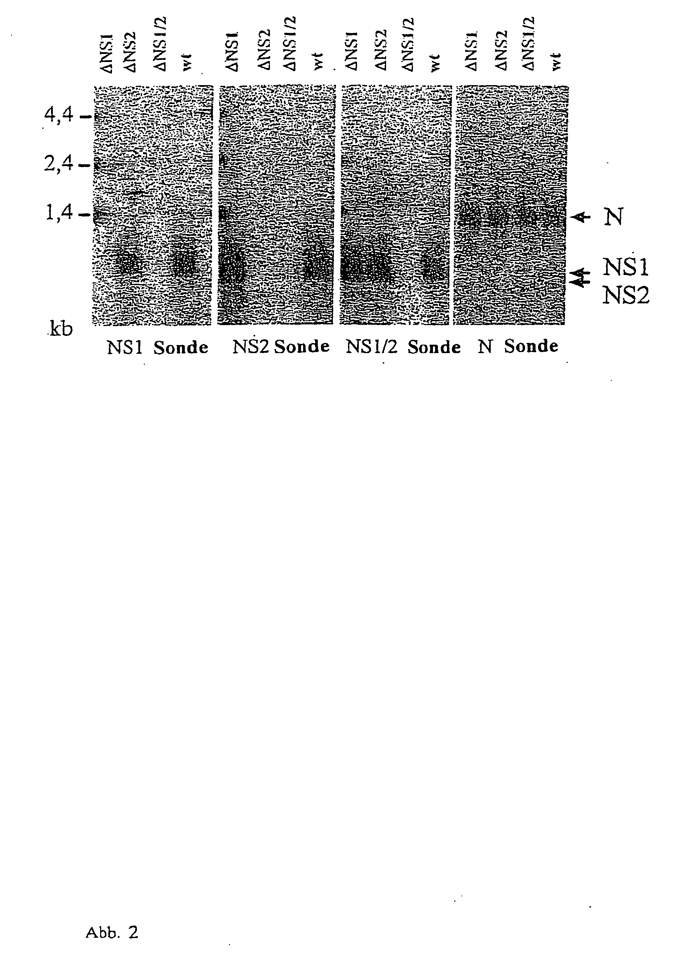

**Abb. 2.** Abwesenheit von NS1 und NS2 Transkripten in den rekombinanten BRSV. Gesamt-RNA aus BSR Zellen, die mit den angegebenen Viren infiziert waren, wurde 2 bis 4 Tage nach der Infektion isoliert und durch Northern-Hybridisierung mit Sonden, die die NS1, NS2, NS1 bis NS2, und N Gene umfassen, analysiert. Die NS1, NS2 und N mRNAs sind markiert.

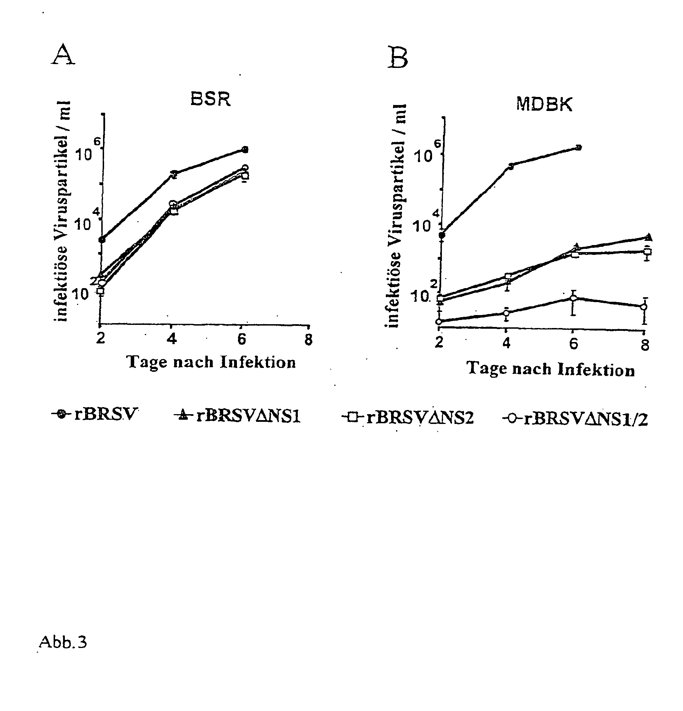

**Abb. 3**. NS-Deletions Mutanten sind in MDKB-Zellen stärker attenuiert als in BSR Zellen. Beinahe konfluente BSR T7-5 (A) und MDBK (B) Zellrasen wurden mit einer MOI von 0,01 mit BRSV (schwarze Kreise), BRSV ΔNS1 (weiße Quadrate), BRSV Δ NS2 (schwarze Dreiecke), oder BRSV ΔNS1/2 (weiße Kreise) infiziert. Die infektiösen Virustiter wurden alle zwei Tage wie im Beispielsteil beschrieben bestimmt. Ab dem sechsten Tag führte in den BSR Zellen die Replikation aller Mutanten und in den MDKB-Zellen die Replikation des wt BRSV zu massiver Zellzerstörung. Die Werte stammen von zwei unabhängigen Experimenten, die jeweils dreifach durchgeführt wurden. Die Balken zeigen die Standardabweichung an.

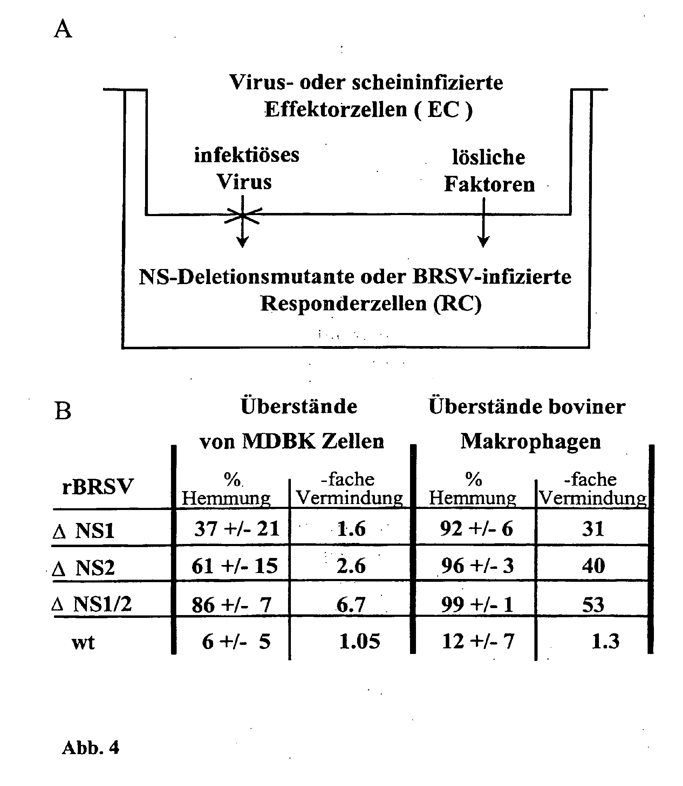

**Abb. 4.** Überstände von Virus-infizierten MDKB-Zellen oder infizierten Makrophagen hemmen das Wachstum der BRSV NS Deletionsmutanten in kokultivierten Vero-Zellen. Das Design der Kokultivierungsexperimente ist schematisch in (A) gezeigt. MDKB-Zellen oder LPS-stimulierte bovine Makrophagen wurden mit BRSV bei einer MOI von 1 infiziert, in Nunc "Anopore Membrane" Zellkultur Einsätze ausgesät, und mit Vero "Responder" Zellen, die mit wt BRSV, BRSV ΔNS1, BRSV ΔNS2, oder BRSV ΔNS1/2 infiziert waren, kultiviert. Nach drei Tagen wurden die Einsätze entfernt und die infektiösen Virustiter der Vero-Zellen bestimmt. Die Ergebnisse in (B) sind angegeben als % Hemmung inklusive Standard-Abweichung sowie als -fache Reduktion (ausgehend vom Durchschnitt) gegenüber Kontrollen, bei denen nicht infizierte MDBK oder nicht infizierte, nicht stimulierte Makrophagen eingesetzt wurden. Die Werte stammen von 6 (MDBK) und 4 (Makrophagen) Kokultivierungsexperimenten.

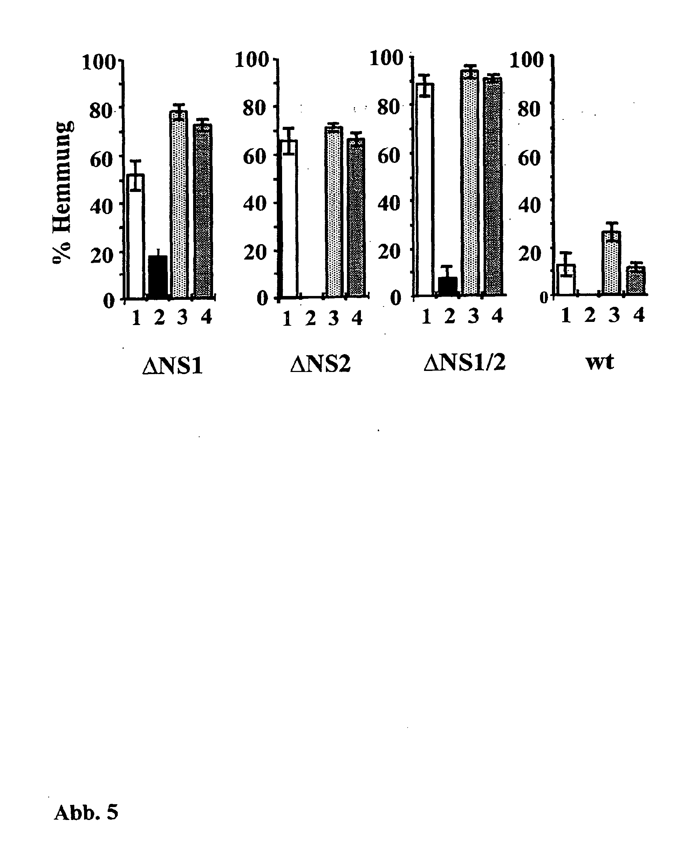

**Abb. 5**. Ein Interferon alpha Rezeptor (IFNA-R2) monoklonaler Antikörper (#2) neutralisiert die Wirkung des von MDBK oder Makrophagen produzierten hemmenden Faktors. Vero "Responder" Zellen, die mit einer MOI von 0.1 mit rBRSV ΔNS1, rBRSV ΔNS2, rBRSV ΔNS1/2 oder wt rBRSV infiziert waren, wurden 3 Stunden lang mit jeweils 5 ug/ml eines monoklonalen Antikörper gegen IFNAR2 (#2), MHC I (#3), TNF-R1 (#4), oder in Abwesenheit von Antikörpern (#1) inkubiert. Die Kokultivierung mit infizierten MDKB-Zellen (siehe Design des Experiments in Abb. 4) wurde in Anwesenheit von 1 ug/ml des entsprechenden Antikörpers durchgeführt. Die Titer wurden in sechs (#1 und #2) oder zwei Experimenten (#3 und #4) bestimmt. Balken geben die Standardabweichung an.

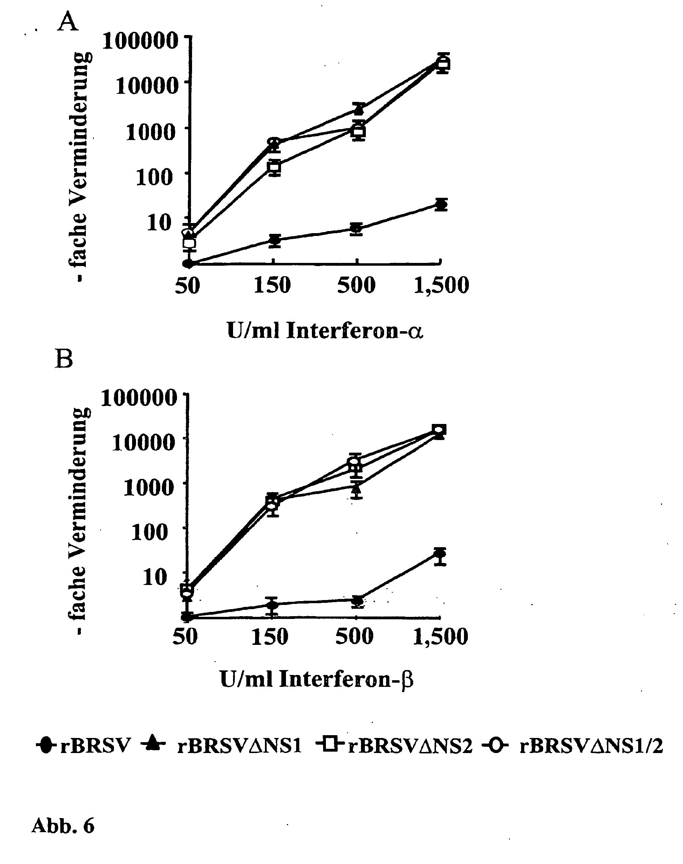

**Abb. 6.** Alle BRSV NS-Deletionsmutanten sind Typ I IFN sensitiv. Vero-Zellen, die mit einer MOI von 0,1 mit BRSV (schwarze Kreise), BRSV ΔNS1 (weiße Quadrate), BRSV ΔNS2 (Dreiecke), oder BRSV ΔNS1/2 (weiße Kreise) infiziert waren, wurden mit den angegebenen Mengen an rekombinantem IFN alpha A/D (A) oder IFN beta (B) inkubiert. Infektiöse Virustiter wurden 4 Tage nach der Infektion bestimmt.

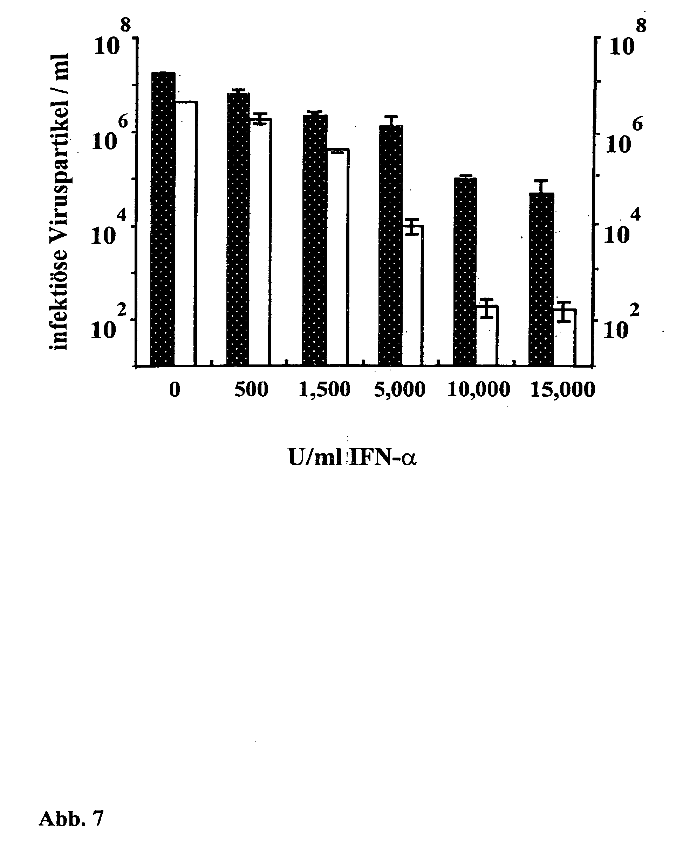

**Abb. 7**. Die IFN Resistenz von BRSV ist in bovinen Zellen besser ausgeprägt als in Primatenzellen. MDBK (gefüllte Säulen) oder Vero-Zellen (offene Säulen) wurden mit einer MOI von 1 mit rBRSV infiziert und mit den angegebenen Mengen rekombinantem IFN alpha A/D behandelt. Infektiöse Virustiter wurden 3 Tage nach der Infektion bestimmt.

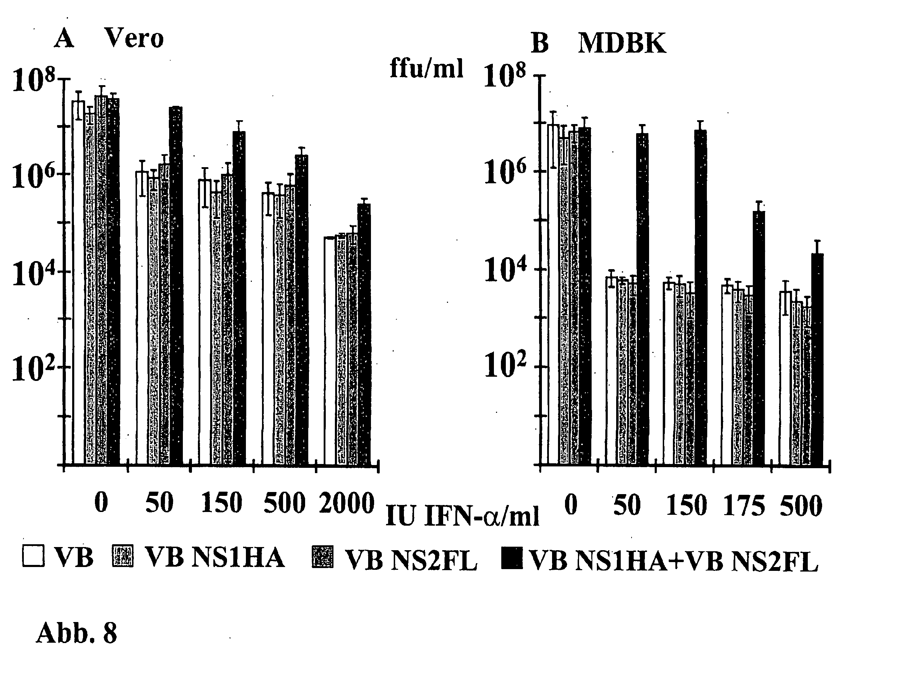

**Abb. 8**. Erhöhte IFN-Resistenz von Tollwutviren (rabies virus; RV) in Zellen, die mit NS1 und NS2-exprimierenden RVs infiziert wurden. Vero (A) oder MDKB-Zellen (B) wurden mit RV SAD VB, SAD VB-NS1, oder SAD VB-NS2, infiziert, oder mit SAD VB-NS1 und SAD VB-NS2 ko-infiziert. Unmittelbar nach der Infektion wurden die Kulturen mit den angegebenen Mengen IFN alpha A/D behandelt. Infektiöse Virustiter wurden 2 Tage nach der Infektion bestimmt. Die Ergebnisse repräsentieren die Mittelwerte aus mindestens vier unabhängigen Experimenten und die Fehlerbalken geben die Standardabweichung an.

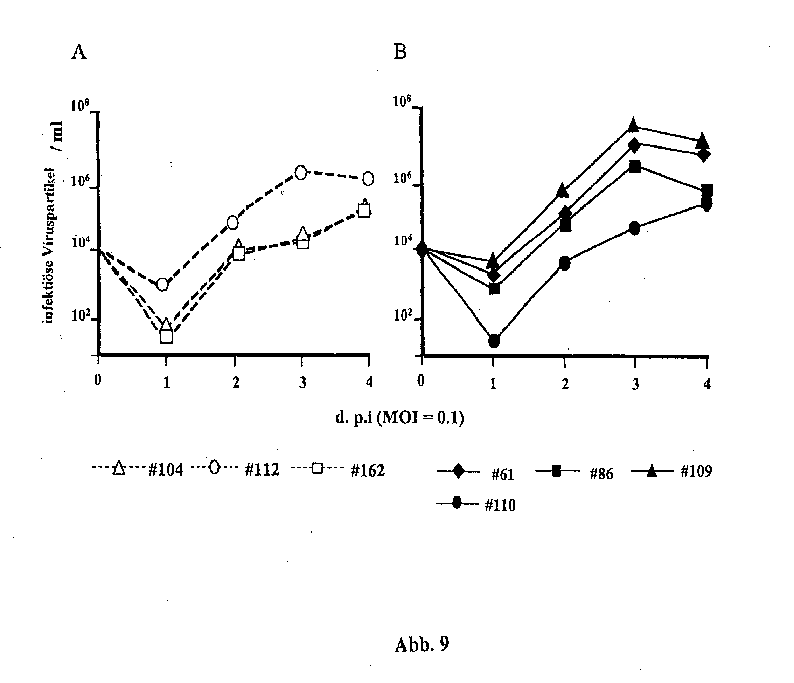

**Abb. 9**. Jeweils 1x10⁵ Hep2 Zellen wurden in Doppelansätzen mit den verschiedenen HRSV-Isolaten für 1 Stunde (MOI=0.1) in 0.5 ml DMEM w/o FCS infiziert, wonach jeweils 0.5 ml + 5% FCS hinzugegeben wurden. Anschließend wurden die infizierten Zellen in 4 cm² Gewebekulturschalen ausgesät. Nach 1, 2, 3 und 4 Tagen wurde für jedes Virusisolat ein Zeitwert entnommen, das Virus durch Frieren/Tauen freigesetzt und der Titer der verschiedenen klinischen Isolate in Abhängigkeit von der Infektionsdauer durch Titration bestimmt. Die pfu/ml wurden nach Anfärben der infizierten Zellen mit einem Antikörper gegen RSV-F durch Auszählen bestimmt.

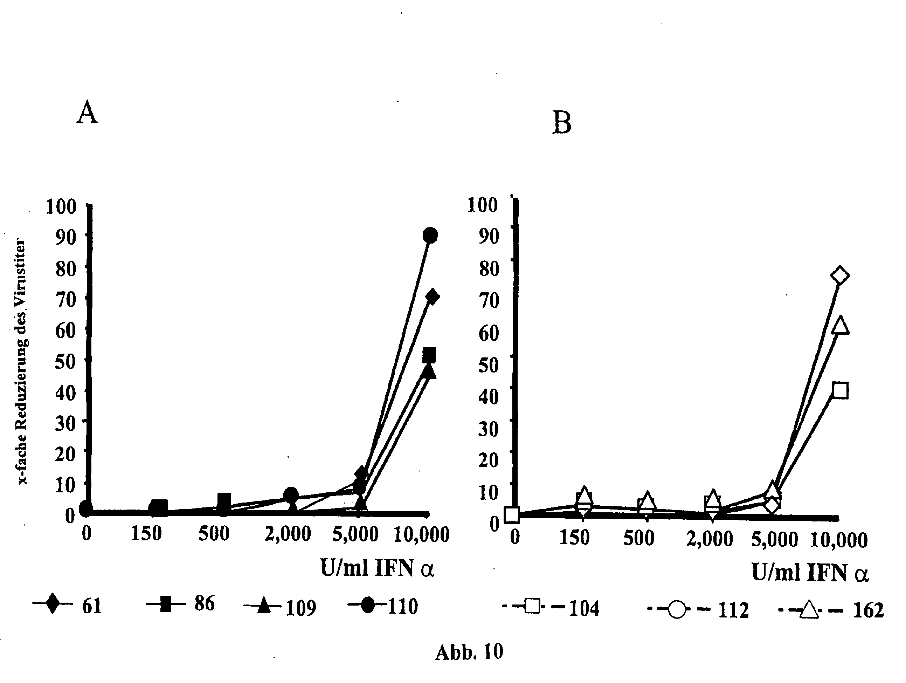

**Abb. 10.** Jeweils 1x10⁵ Hep2 Zellen wurden in Doppelansätzen mit den verschiedenen HRSV-Isolaten für 1 Stunde (MOI=0.1) in 0.5 ml DMEM w/o FCS infiziert und anschließend in 4 cm² Gewebekulturschalen ausgesät. 0, 150, 500, 10,000 U/ml rekombinantes Typ I IFN A/D wurden in weiteren 0,5 ml DMEM + 5% FCS aufgenommen und nach 30 min hinzugegeben.
Nach 72 Stunden Inkubationsdauer wurde das Virus durch Frieren/Tauen freigesetzt und der Titer der verschiedenen klinischen Isolate in Abhängigkeit der applizierten IFN-Menge durch Titration bestimmt. Die pfu/ml wurden nach Anfärben der infizierten Zellen mit einem Antikörper gegen RSV-F durch Auszählen bestimmt.

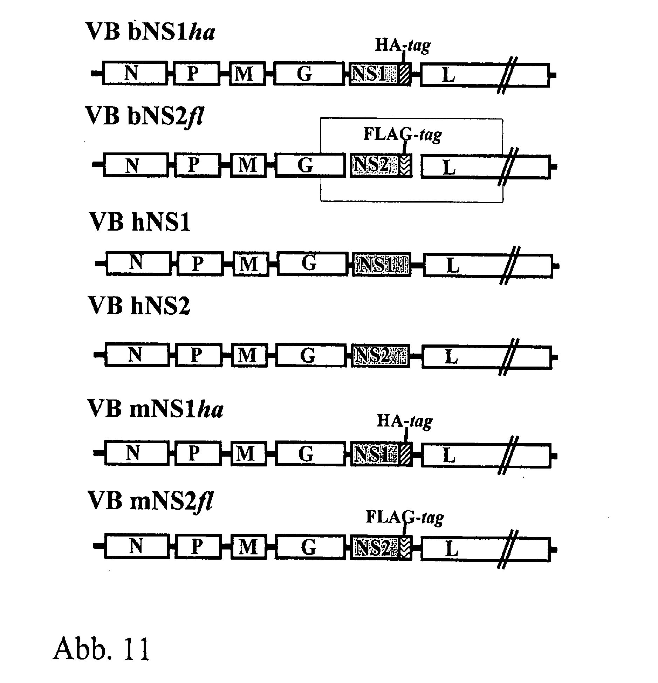

**Abb. 11**. Organisation der rekombinanten Tollwutviren (RV), die HRSV NS1 oder HRSV NS2 oder genetisch markierte BRSV NS1, BRSV NS2, PVM NS 1 oder PVM NS2 ORFs zwischen den RV G und L Genen besitzen.

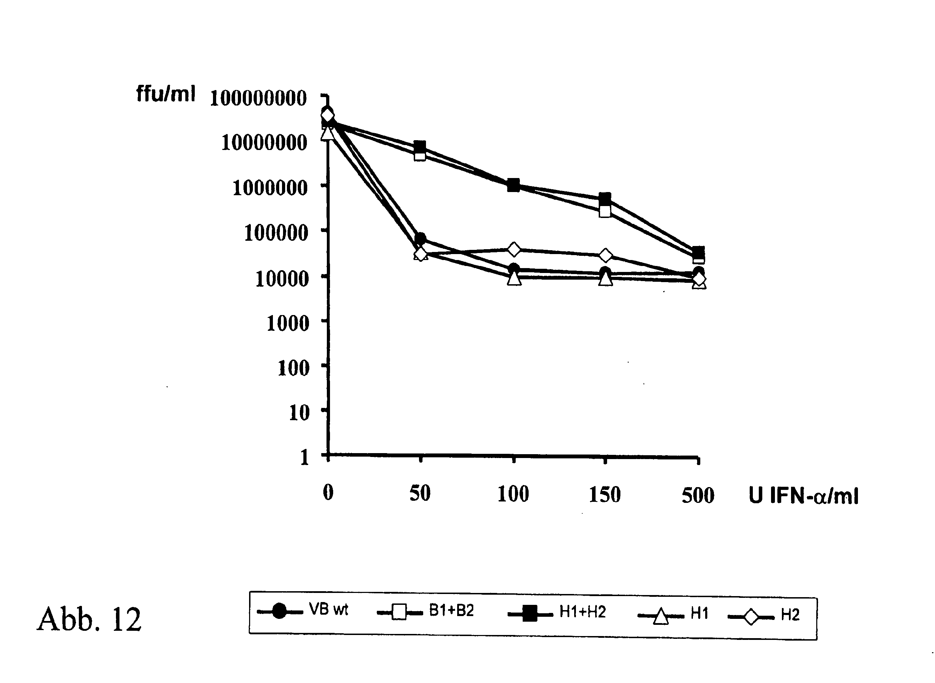

**Abb. 12.** Erhöhte IFN-Resistenz von Tollwutviren (rabies virus; RV), die mit NS1 und NS2-exprimierenden RVs infiziert wurden. MDKB-Zellen wurden mit RV SAD VB, SAD VB-HNS1, oder SAD VB-hNS2 infiziert, oder mit SAD VB-hNS 1 und SAD VB-hNS2 oder SAD VB-BNS1 und SAD VB-bNS2 ko-infiziert. Unmittelbar nach der Infektion wurden die Kulturen mit den angegebenen Mengen IFN alpha A/D behandelt. Infektiöse Virustiter wurden 2 Tage nach der Infektion bestimmt. Die Ergebnisse repräsentieren die Mittelwerte aus vier unabhängigen Experimenten und die Fehlerbalken geben die Standardabweichung an.

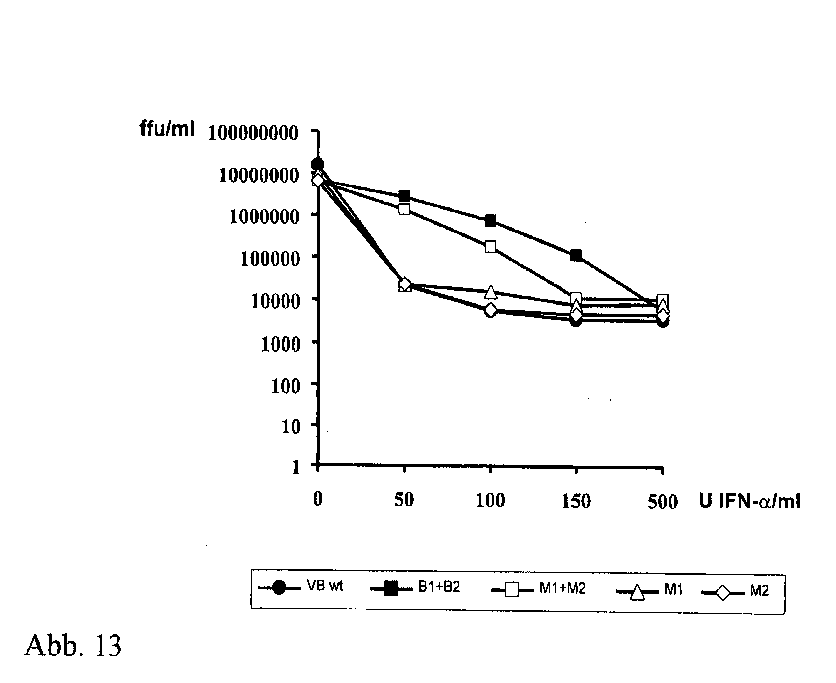

**Abb. 13.** Erhöhte IFN-Resistenz von Tollwutviren (rabies virus; RV), die mit NS1 und NS2-exprimierenden RVs infiziert wurden. MDKB-Zellen wurden mit RV SAD VB, SAD VB-mNS1, oder SAD VB-mNS2 infiziert, oder mit SAD VB-mNS 1 und SAD VB-mNS2 oder SAD VB-bNS1 und SAD VB-bNS2 ko-infiziert. Unmittelbar nach der Infektion wurden die Kulturen mit den angegebenen Mengen IFN alpha A/D behandelt. Infektiöse Virustiter wurden 2 Tage nach der Infektion bestimmt. Die Ergebnisse repräsentieren die Mittelwerte aus vier unabhängigen Experimenten und die Fehlerbalken geben die Standardabweichung an.

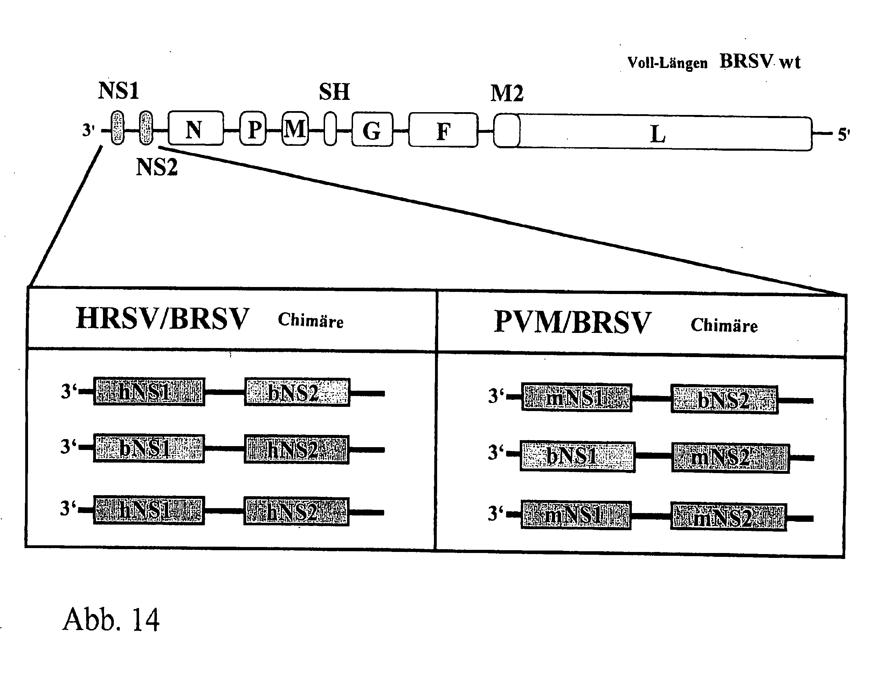

**Abb. 14.** Organisation der chimären BRSVs mit heterologen NS Genen.

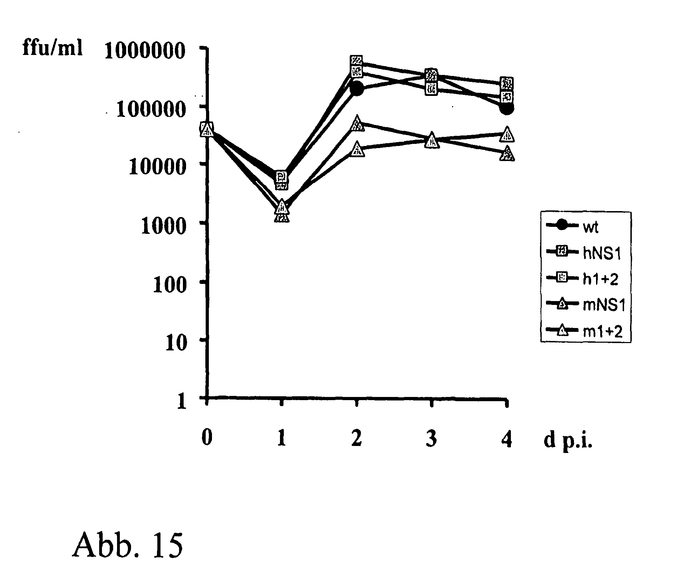

**Abb. 15**. Die PVM/BRSV Chimären sind auf Vero-Zellen leicht attenuiert. Vero-Zellen wurden mit einer MOI von 0.1 mit BRSV wt (schwarze Kreise), BRSV hNS1bNS2 (dunkelgraue Quadrate), BRSV hNS1hNS2 (hellgraue Quadrate), BRSV mNS1bNS2 (dunkelgraue Dreiecke) und BRSV mNS1mNS2 (hellgraue Dreiecke) infiziert. Die infektiösen Virustiter wurden an vier aufeinanderfolgenden Tagen bestimmt. Die Werte stammen von mindestens zwei unabhängigen Experimenten.

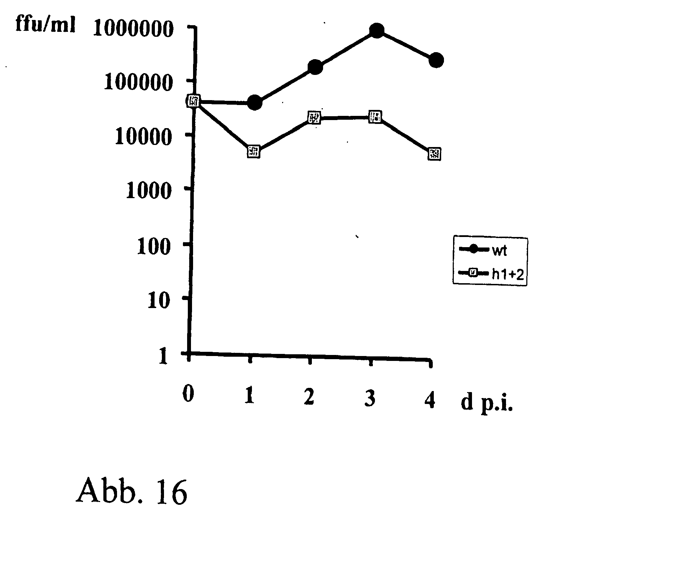

**Abb. 16.** Das Wachstum von BRSV hNS1hNS2 ist auf MDKB-Zellen attenuiert. MDKB-Zellen wurden mit einer MOI von 0.1 mit BRSV wt (schwarze Kreise) und BRSV hNS1hNS2 (hellgraue Quadrate) infiziert. Die infektiösen Virustiter wurden an vier aufeinanderfolgenden Tagen bestimmt. Die Werte sind Mittelwerte von mindestens zwei unabhängigen Experimenten.

**Abb**. **17**. Auf MDKB-Zellen ist BRSV hNS1hNS2 IFN TypI sensitiv. Vero (A) und MDBK (B) Zellen wurden mit einer MOI von 0.1 mit BRSV wt (schwarze Kreise) und BRSV hNS1hNS2 (hellgraue Quadrate) infiziert und anschließend mit den angegebenen Mengen an rekombinantem IFN alpha A/D inkubiert. Infektiöse Virustiter wurden 3 Tage nach Infektion bestimmt. Die Werte stammen von mindestens zwei unabhängigen Experimenten.

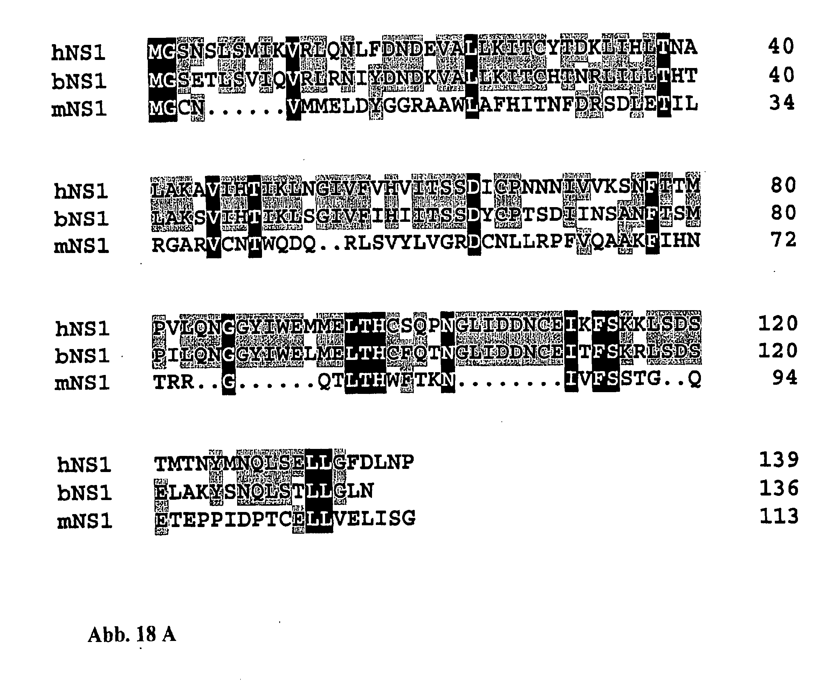

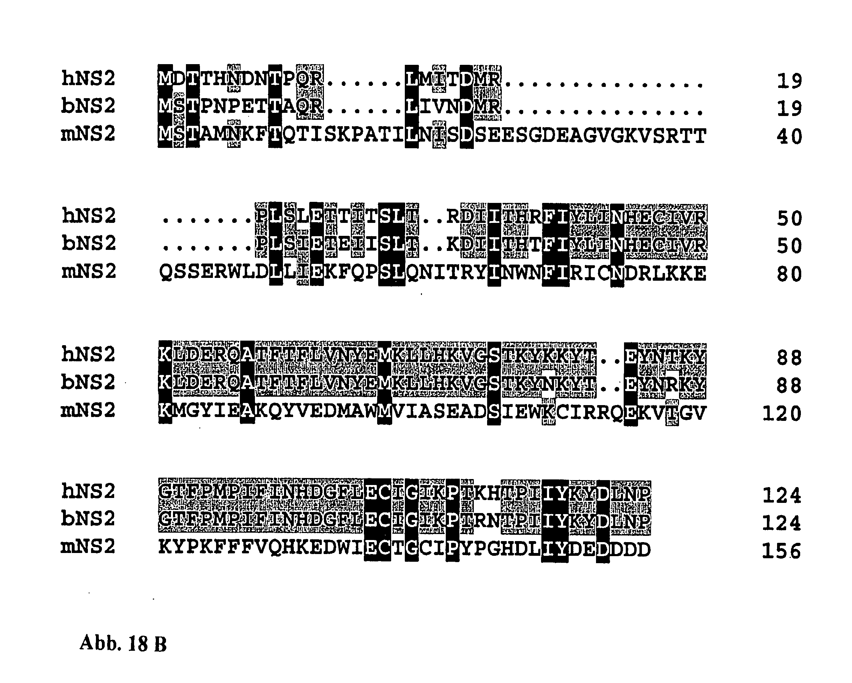

**Abb. 18.** Vergleich der Aminosäuresequenz der NS1 (Abb. 18A) und NS2 Proteine (Abb. 18B) von HRSV (hNS1; hNS2), BRSV (bNS1, bNS2) und PVM (mNS1, mNS2); GenBank Accession No:U35030 (HRSV Stamm Long), AF092942 (BRSV Stamm ATue 51908) und D 10331 (PVM).

Die folgenden Beispiele werden zum Zweck der Veranschaulichung verschiedener Ausführungsformen der Erfindung aufgezeigt und sind nicht gedacht, die vorliegende Erfindung in irgendeiner Weise einzuschränken.

### BEISPIELE

### Beispiel 1: Konstruktion und Rescue von BRSV Deletionsmutanten ohne NS-Gene

Das rekombinante BRSV (rBRSV) stammt vom BRSV Stamm A51908 (American Type Culture Collection (ATCC)) (33), Variante Atue51908 (GenBank Acc. Nr. AF092942) und wurde in MDKB-Zellen angezogen wie bereits beschrieben (5). Die Klonierung der Gesamtlänge-cDNA von BRSV Stamm Atue51908 (GenBank Acc. Nr. AF092942) und die Konstruktion der Plasmide, die die T7 RNA Polymerase-vermittelte Transkription der BRSV Gesamtlängen-antigenomischen RNA oder der antigenomischen RNA ohne NS2 Gen (pBRSV ΔNS2) erlauben, wurde bereits beschrieben (5).

Die Konstrukte, denen das NS1 Gen (pBRSV ΔNS1) oder die beiden Gene NS1 und NS2 fehlen (pBRSV ΔNS1/2), wurden ebenfalls auf der Basis von pBRSV erzeugt. Das NS1 Gen wurde aus pBRSV entfernt, indem mit *Not*I und *Ase*I geschnitten, mit Klenow Polymerase aufgefüllt und anschließend religiert wurde. Daraus entstand pBRSV ΔNS1. Für die Herstellung der Doppeldeletionsmutante pBRSV ΔNS1/2 wurde ein 0.6 kb PCR Fragment amplifiziert, das Teil des N Gens umfaßt. Dazu wurde der Primer NNot(+) (5'-TAGGCGGCCGCAAAAATGGCTCTTAGCAAGGTG-3') verwendet, der eine *Not*I Schnittstelle (unterstrichen) enthält, die stromaufwärts des N Startcodons liegt, sowie der reverse Primer Nstu(-)(5'-TCCTTTGTATCGTTTCATTTC-3'), der den nt 1735-1715 von rBRSV entspricht, die stromabwärts der einzigen StuI Schnittstelle liegen (rBRSV Position 1671). Nach Deletion der NS1 und NS2 Gene und eines Teils des N Gens aus pBRSV durch Verdau mit *Not*I (rBRSV Position 72) und *Stu*I (rBRSV Position 1671) wurde das StuI-verdaute PCR Fragment benutzt, um die deletierten Sequenzen zu ersetzen (Fig. 1).

Im Vergleich zu der Sequenz des rekombinanten wt Virus rBRSV fehlen den NS1, NS2 und NS1/2 Deletionsmutanten 496, 509 bzw. 1060 Nukleotide. In allen Konstrukten wird die Transkription des 3' terminalen Gens durch das ursprüngliche leader/NS1 Transkriptionsstartsignal eingeleitet (Fig. 1).

Lebensfähige rekombinante Viren rBRSV, rBRSV ΔNS1, rBRSV ΔNS2 und rBRSV Δ NS1/2 konnten von den jeweiligen cDNA-Konstrukten in T7 RNA Polymerase exprimierenden BSR T7/5 Zellen (5) nach Transfektion (CaPO₄ Protokoll; Mammalian Transfection Kit, Stratagene) von T7 Promotor-kontrollierten Plasmiden mit der jeweiligen Virus-cDNA (10 µg) gewonnen werden. Plasmide, die für die BRSV Proteine N und P (pTITB-N und pTITB-P, 4 µg pro Plasmid) und L und M2 (pTITB-L and pTITB-M2, 2 µg pro Plasmid) kodieren, wurden mit der jeweiligen Virus-cDNA in ungefähr 10⁶ BSR T7/5 Zellen, die die RNA Polymerase des Phagen T7 stabil exprimieren (5), ko-transfiziert. Nach 4 Stunden wurde das Transfektionsmedium abgenommen und BHK-21 Medium (Gibco) mit 5% FCS zugegeben. Mit BRSV cDNA transfizierte Zellen wurden alle 5 Tage im Verhältnis 1:3 umgesetzt bis ein cytopathogener Effekt sichtbar wurde.

In allen Fällen, einschließlich der NS1/2 Doppeldeletionsmutante, resultierte die Ko-Transfektion der für die BRSV Proteine N, P, L und M2 kodierenden Support-Plasmide in der Bildung von Syncytien. Die Viren wurden nach Umsetzen der transfizierten Zellen im Verhältnis 1:3 und dem Auftreten eines deutlichen cytopathogenen Effekts geerntet.

Für die Herstellung von Virus-Stammlösungen wurden zu 80% konfluente MDBK und Vero-Zellen mit einer Multiplizität der Infektion (MOI) von 0.1 in Serum-freiem Dulbecco's minimal essential medium (DMEM) infiziert. Nach einstündiger Adsorption wurde das Inoculum entfernt und die Zellen bei 37°C in DMEM, das mit 2,5% FCS ergänzt war, in einer 5%-igen CO₂-Atmosphäre inkubiert bis ein deutlicher cytopathischer Effekt (CPE) zu sehen war. Die Viren wurden durch Frieren und anschließendes Auftauen freigesetzt. Die Virustiter wurden auf Vero-Zellen durch Verdünnungsreihen in Mikrowell-Platten und anschließendem Auszählen der infizierten Foci bestimmt. Dabei wurden die Foci durch indirekte Färbung mit einem Antikörper gegen das Fusionsprotein F angefärbt (freundlicherweise von J.A. Melero, Madrid, zur Verfügung gestellt). Die Herstellung der Virus-Stammlösungen der NS Deletionsmutanten erfolgte auf Vero-Zellen, die mit einer MOI von 0.01 infiziert wurden. Nach Infektion von Vero-Zellen mit einer MOI von 0.1 dauerte es 3 Tage bei rBSRV und 5 Tage bei den NS Deletionsmutanten, bis ein deutlicher CPE zu sehen war.

### Beispiel 2: Wachstum der NS Deletionsmutanten

Die Wachstumscharakeristika der Viren wurden zunächst in der von BHK-Zellen abgeleiteten Zellinie BSR T7/5 analysiert, die für den Rescue der Viren verwendet wurde. Im Vergleich zum parentalen Gesamtlängen-Virus waren alle drei Mutanten attenuiert, was auf einen Beitrag beider NS Proteine zur Virusreplikation hinweist. Interessanterweise konnten dabei keine offensichtlichen Unterschiede in der Verbreitung der Viren in infizierten Zellen und in den Endtitern zwischen den zwei Einzel- und der Doppeldeletionsmutante festgestellt werden. Alle Mutanten erreichten infektiöse Titer von 2x10⁵ pfu nach Infektion von BSR T7/5 Zellen mit einer MOI von 0.1 und anschließender Inkubationszeit von 6 Tagen. Im Gegensatz dazu erreichte das parentale Virus bis zu 1x10⁶pfu (Fig. 3A). Ähnliche Ergebnisse mit leicht erhöhten Titern wurden durch Infektion von Hep2 oder Vero-Zellen erreicht, wobei letztere die bevorzugte Zell-Linie für die Kultivierung von HRSV darstellt.

Anschließend wurde eine Zellinie bovinen Ursprungs, MDBK, verwendet, die das Wachstum des wt BRSV optimal unterstützt (5). Tatsächlich konnten in MDBK leicht erhöhte wt BRSV Titer von 2x10⁶ pfu nach 6 Tagen Infektion erreicht werden (Fig. 3B). Erstaunlicherweise war das Wachstum der Deletionsmutanten in dieser Zellinie allerdings stark beeinträchtigt. Die Einzeldeletionsmutanten ΔNS1 und ΔNS2 erreichten Titer von nur 3x10³ pfu nach 6 Tagen, also 100-fach niedriger als in BSR Zellen. Die Doppeldeletionsmutante ΔNS1/2 war nicht in der Lage, sich in den ersten 6 Tagen der Infektion bemerkenswert zu vervielfältigen. Erst nach Umsetzen der Zellen und einer weiteren Inkubation von 8 Tagen konnten Virustiter von 2x10² erhalten werden. Obwohl MDKB-Zellen die optimale Wirtszelle für wt BRSV darstellen, sind sie offensichtlich fast nicht permissiv für alle NS Deletionsmutanten, während hingegen BSR und Vero-Zellen, die suboptimale Wirtszellen für wt BRSV darstellen, das Wachstum der NS Deletionsmutanten relativ gut unterstützten.

### Beispiel 3: Northern Hybridisierung mit RNA von BRSV Deletionsmutanten ohne NS Gene

Für Northern Hybridisierungen wurde die RNA aus Zellen isoliert, die mit rBRSV oder den NS Deletionsmutanten infiziert waren.

Vero-Zellen wurden mit einer MOI von 0.1 mit den rekombinanten Viren rBRSV, rBRSV ΔNS1, rBRSV ΔNS2 und rBRSV ΔNS1/2 infiziert und Gesamt-RNA wurde isoliert, nachdem ein starker CPE zu beobachten war (für rBRSV nach 3 Tagen, für NS Deletionsmutanten nach 5 Tagen). Die RNA wurde durch denaturierende Gelelektrophorese aufgetrennt, auf eine Nylonmembran transferiert (Duralon-UV, Stratagene) und mittels UV-Strahlung auf die Membran gebunden. NS1, NS2 und N Gen-spezifische DNA-Proben von ungefähr 500 nt Länge wurden durch Nick Translation mit (α-³²P)dCTP (3,000 Ci/mmol, Amersham) markiert (Nick-translation kit, Amersham). Mit den hybridisierten Filter wurden unter Verwendung von intensivierenden Screens Kodak BioMax MS Filme belichtet, oder sie wurden mittels Phosphorimaging (Storm, Molecular Dynamics) ausgewertet.

Für alle Viren wurden ähnliche Transkript-Muster festgestellt, wobei sich die Viren nur in der An- oder Abwesenheit der NS1- und NS2-spezifischen RNAs unterschieden (Fig. 2). Wie bereits für die BRSV und HRSV NS2 Deletionsmutanten beobachtet (5;43), waren ähnliche Mengen an N-mRNA und genomischer RNA in allen infizierten Zellen vorhanden, unabhängig von der verwendeten Rekombinante. Demnach wirken die NS Proteine eher auf RNA Synthese im allgemeinen, als bestimmte Schritte der RNA Replikation oder Transkription zu beeinflussen.

### Beispiel 4: Lösliche Faktoren, die von MDKB-Zellen und bovinen Makrophagen produziert werden, beeinflussen das Wachstum der NS Deletionsmutanten

Um zelluläre Faktoren zu identifizieren, die für die offensichtlich selektive Behinderung des Wachstums der NS Deletionsmutanten in MDKB-Zellen verantwortlich sind, wurde als erstes geprüft, ob lösliche Moleküle, die von MDKB-Zellen produziert werden, in der Lage sind, das Wachstum der NS Deletionsmutanten einzuschränken. Dazu wurden MDBK und Vero-Zellen in Vorrichtungen kokultiviert, die es ermöglichen, die beiden Zellkulturen durch eine virusundurchlässige, jedoch für lösliche Faktoren durchlässige Membran zu trennen (Fig. 4A). In der oberen Schale wurden MDKB-Zellen als Effektorzellen benutzt, während Vero-Zellen in der unteren Schale als Responderzellen dienten.

Vero Responderzellen wurden in Suspension entweder mock-infiziert oder mit rBRSV ΔNS1, rBRSV ΔNS2 oder rBRSV ΔNS1/2 mit einer MOI von 0.1 eine Stunde lang in DMEM ohne FCS infiziert. Nach dem Waschen wurden jeweils 5x10⁵ Zellen in DMEM mit 2,5% FCS in 6-well Schälchen ausgesät. MDBK Effektorzellen oder bovine Makrophagen, die über Nacht mit 10 µg/ml LPS (Sigma) stimuliert wurden, wurden in Suspension für 1hr mit einer MOI von 1 mit rBRSV infiziert. Nach dem Waschen wurden 1x10⁶ Zellen in 25 mm Zellkultureinsätzen ausgesät, die mit einer 200 nm Anopore-Membran (Nunc) versehen waren, und in die Schälchen mit den infizierten Vero "Responder" Zellen gesetzt. Nach einer dreitägigen Kokultivierung wurden die Membraneinsätze entfernt und die Virustiter der Vero-Zellen, wie in Beispiel 1 beschrieben, bestimmt.

Mindestens fünf unabhängige Kokultivierungsversuche wurden durchgeführt. Nichtinfizierte MDBK Effektorzellen oder als Negativkontrolle verwendete BSR Zellen zeigten keinen hemmenden Effekt auf das Wachstum des wt BRSV oder der NS Deletionsmutanten in den Vero Responderzellen. Kokultivierung mit BRSV-infizierten MDKB-Zellen (MOI = 1) resultierte in einer schwachen, aber reproduzierbaren Hemmung der NS Deletionsmutanten, während hingegen das Wachstum des wt BRSV nicht beeinträchtigt war (Fig. 4B). Der deutlichste Effekt konnte mit der NS1/2 Doppeldeletionsmutante beobachtet werden. Hier waren die Titer ungefähr 7-fach reduziert in der Gegenwart von infizierten MDKB-Zellen, verglichen mit nichtinfizierten MDKB-Zellen. Die Titer der Einzeldeletionsmutanten rBRSV DNS1 and rBRSV ΔNS2 waren in diesem Fall 2- bzw. 4-fach reduziert.

Da die Überstände von nichtinfizierten MDKB-Zellen nicht in der Lage waren, das Wachstum der Deletionsmutanten in Vero Responderzellen zu beeinträchtigen, wurde angenommen, daß die wirksamen MDBK Faktoren durch die Virusinfektion induziert wurden. Nicht nur Infektionen mit wt BRSV, sondern auch mit einer Reihe von BRSV Deletionsmutanten, unter anderem rBRSV ΔNS1/2 und einer Mutante, die das SH- und G-Gen deletiert hatte (rBRSV ΔSH/G; nicht publiziert), führten zu der Sekretion der wirksamen Faktoren. Zusätzlich konnte gezeigt werden, daß die Infektion mit einem anderen RNA Virus, Rabies Virus, auch zur Induktion der einwirkenden Faktoren in MDKB-Zellen führte. Diese Ergebnisse wiesen stark auf eine Cytokin-vermittelte, insbesondere durch Interferon, vermittelte Induktion des antiviralen Zustands in Vero Responder Zellen hin.

Um die beteiligten Cytokine näher zu untersuchen, wurden bovine Makrophagen als Effektorzellen verwendet. Bovine Makrophagen wurden aus dem Blut einer Kuh und eines Kalbs mittels Ficoll Gradientenzentrifugation (Lymphoflot, Biotest, Dreieich) bei 1,500 rpm und Adsorption der mononukleären Zellfraktion an den Boden der Zellkulturflasche isoliert. Nach einer Übernachtinkubation wurden die nichtadhärenten Zellen durch dreimaliges Waschen mit RPMI (Gibco) ohne FCS entfernt. Die übrigen, adhärenten Zellen (90-95% positiv für CD14) wurden in RPMI mit 10% FCS bei 37°C und 5% CO₂ inkubiert. Isolierte bovine Makrophagen wurden über Nacht mit LPS stimuliert, mit rBRSV infiziert und, wie oben beschrieben, mit Vero-Zellen kokultiviert.

Die erhaltenen Mengen an Gesamtlängen-rBRSV waren nach Inkubation mit stimulierten, virusinfizierten oder nichtstimulierten Makrophagen unverändert. Im Vergleich mit der nichtstimulierten und nichtinfizierten Makrophagenkontrolle konnte für die NS Deletionsmutanten jedoch eine 30- bis 50-fache Reduktion beobachtet werden (Fig. 4B). Auch die alleinige Stimulation der Makrophagen mit LPS ohne nachfolgende Virusinfektion war ausreichend, um eine ungefähr 10-fache Reduktion von rBRSV ΔNS1/2 zu verursachen. Da stimulierte Makrophagen als Produzenten von Typ I Interferon bekannt sind, deuteten diese Experimente auf eine Beteiligung von IFN alpha und/oder beta bei der Hemmung des Wachstums der BRSV NS Deletionsmutanten hin.

### Beispiel 5: Die Deletion der NS Gene macht BRSV sensitiv für Typ I Interferon

Mittels FACS Analyse konnte nachgewiesen werden, daß die in Ko-Kultivierungsversuchen (siehe Beispiel 4) als Responderzellen verwendeten Vero-Zellen die alpha-Untereinheit des Interferon Typ I Rezeptors (IFNAR2) (44) exprimieren. Um herauszufinden, ob das von infizierten MDKB-Zellen oder Makrophagen produzierte IFN alpha und/oder beta den inhibitorischen Effekt auf die NS Deletionsmutanten vermittelt, wurden Vero Responderzellen mit rBRSV ΔNS1/2, rBRSV ΔNS2, or rBRSV ΔNS1/2 infiziert und anschließend mit einem monoklonalen Antikörper behandelt, der den IFNAR2 blockiert (PBL-Laboratories). Die Behandlung der Responderzellen erfolgte unmittelbar nach der Infektion, indem die Zellen 1 hr mit 5 µg/ml eines neutralisierenden Maus anti-human Interferon alpha/beta Rezeptorkette 2 (CD118) Antikörper (PBL Biomedical Laboratories), oder mit 5 µg/ml eines Kontrollantikörpers, der den TNFRI oder MHC Klasse I Moleküle erkannte, inkubiert wurden. Nach dem Umsetzen der Zellen in six-well Schälchen wurden die Zellen in 1 µg/ml des jeweiligen Antikörpers gehalten und in der Anwesenheit von infizierten MDBK Effektorzellen für drei Tage inkubiert.

Während in den Zellkulturen mit den Kontrollantikörpern oder ohne Antikörper eine Inhibition der NS Deletionsmutanten zu beobachten war, war der inhibitorische Effekt in den Zellen, die mit dem IFNAR2 Antikörper behandelt wurden, fast vollständig aufgehoben (Fig. 5). Dadurch ließ sich die Induktion eines antiviralen Zustands in Vero Responderzellen alleinig auf die von MDKB-Zellen oder Makrophagen produzierten Typ I Interferone zurückführen.

Rekombinante humanes Typ I Interferone wurden dann eingesetzt, um das Verhalten des wt und der BRSV Mutanten in IFN-stimulierten Zellen direkt zu analysieren. Um den Effekt des Typ I Interferons auf die Replikation von BRSV und den NS Deletionsmutanten zu untersuchen, wurden Vero oder MDKB-Zellen wie oben beschrieben mit den verschiedenen Viren mit einer MOI von 0.1 infiziert und in six-well Schälchen in DMEM mit 2.5% FCS ausgesät. Rekombinantes universelles Typ I Interferon (humanes Interferon alpha A/D) oder humanes Interferon beta (PBL-Biomedical-Laboratories) wurde bis zu einer Konzentration von 15000 U/ml direkt nach dem Aussäen zugegeben. Die Virustiter wurden nach einer dreitägigen Inkubationszeit durch Verdünnungsreihen und indirektem Anfärben der infizierten Zellfoci mit einem Antikörper gegen das Fusionsprotein F bestimmt.

Alle drei NS Deletionsmutanten zeigten eine sehr ähnliche, starke und dosisabhängige Anfälligkeit gegenüber der durch IFN induzierten zellulären Antwort, wobei 1500 U zu einer mehr als 10000-fachen Reduktion der infektiösen Titer führte (Fig. 6). Im Gegensatz dazu zeigte sich das wt BRSV relativ resistent gegenüber der IFN Behandlung. Der Schutz war aber nicht vollständig und 1500 U IFN alpha oder IFN beta verursachten eine ungefähr 13-fache Reduktion.

Um die Möglichkeit zu prüfen, daß der Schutz des wt BRSV in bovinen Zellen ausgeprägter als in der Primatenzell-Linie Vero ist, infizierten wir in parallelen Experimenten MDBK und Vero-Zellen mit einer MOI von 1 und gaben gleiche Mengen an IFN zu. In unbehandelten MDBK bzw. Vero-Zellen wuchs BRSV zu Titern von 1.7x10⁷ bzw. 4x10⁶ pfu/ml (Fig. 7). Mit IFN Behandlung nahmen die Titer in Vero-Zellen schneller ab als in MDKB-Zellen. Nach der Gabe von 10000 U IFN waren die infektiösen Titer in Vero-Zellen 555-fach niedriger als in MDKB-Zellen, obwohl letztere bereits beträchtliche Zellschäden aufwiesen. Wie durch die starke Inhibition der NS Deletionsmutanten gezeigt wurde, ist die antivirale Antwort der MDKB-Zellen mindestens gleich stark wie die von Vero-Zellen. Daher weist der verstärkte Schutz des wt BRSV in MDKB-Zellen darauf hin, daß BRSV die bovine zelluläre antivirale Antwort effizienter bewältigt als diejenige von Primatenzellen.

### Beispiel 6: BRSV NS1 und NS2 verstärkten gemeinsam die Resistenz von Rabies Virus gegenüber der IFN-vermittelten antiviralen Antwort

Die Deletion jedes NS Gens von BRSV führte zu einem ungefähr gleichen Grad der Sensitivität gegenüber IFN-vermittelten Zellantworten. Dies legt nahe, daß beide NS Proteine nötig sind, den antiviralen Mechanismen entgegenzuwirken. Um die obligatorisch kooperative Funktion von NS1 und NS2 zu bestätigen, und um herauszufinden, ob die beiden NS Proteine zum Schutz eines nichtverwandten Viruses verwendet werden können, haben wir rekombinante Rabies Viren entwickelt, die entweder NS1 (SAD VB-NS1) oder NS2 (SAD VB-NS2) exprimieren. Rekombinante RV mit hinzugefiigtem NS1 oder NS2 Gen (Fig.1) wurden auf der Basis der Gesamtlängen-RV cDNA (SAD L16) konstruiert, die eine zusätzliche Transkriptionsstop- und Restartsequenz in der 3' nichtkodierenden Sequenz des G Gens enthält (SAD VB) (32). Das zusätzliche Gen wurde zwischen dem G und L Gen des attenuierten Rabies Virus SAD L16 (Fig, 1B) eingefügt, eine Methode, die schon für andere Gene erfolgreich durchgeführt wurde (12; 39). Zuerst wurden cDNAs konstruiert, die C-terminal mit einem Protein-tag versehene Versionen der BRSV NS1 oder NS2 Proteine beinhalteten. Direkt vor dem NS 1 Stopkodon wurden mittels PCR unter der Verwendung des reversen Primers NS1HAr-EcoRI (5'GCAATAGAATTCCTAAGCGTAATCTGGTAC ATCATAAGGATAATTCAGACCAAGAAGAGT-3') (enthält eine *EcoRI* Schnittstelle; unterstrichen) 27 zusätzliche Nukleotide eingefügt, die einer internen Region des Influenza Hämagglutinin (HA) Proteins entsprechen. Bei NS2 wurden 24 Nukleotide mit dem reversen Primer NS2FLr-EcoRI (5'-GCAATAGAATTCCTATTTATCGTCATCATCTTTATAATCTGGATTTAAATCAT ACTTATA-3') (*EcoR*I unterstrichen) eingefügt, die dem sythetischen FLAG-Peptid entsprechen. Diese PCR-Fragmente wurden benutzt, um die entsprechenden Sequenzen eines Plasmids zu ersetzen (pBSBRSVNS1NS2), das nt 1 bis nt 957 der Gesamtlängen-cDNA BRSV enthält (5). Das NS1-HA Gen wurde mit *Not*I und *EcoR*I ausgeschnitten. Nach einer Auflüll-Reaktion mit Klenow Polymerase wurde das 475 nt lange Fragment in die einzige *Sma*I Stelle des pSAD VB unmittelbar stromabwärts des zusätzlichen Transkriptionsstartsignals eingefügt. Daraus ergab sich pSAD VB-NS1HA. Ein 470 nt langes NS2-FL Fragment wurde nach dem Ausschneiden mit *Ase*I und *EcoR*I und Auffüllen mit Klenow Polymerase entsprechend kloniert was zu pSAD VB-NS2FL führte.

Rekombinantes RV mit entweder dem NS1 oder dem NS2 Gen konnte wie bereits beschrieben (19) nach Transfektion (CaPO₄ Protokoll; Mammalian Transfection Kit, Stratagene) von T7 Promotor-kontrollierten Plasmiden der jeweiligen Virus cDNA (10 µg) gewonnen werden. Plasmide, die für das RV Protein N (pTIT-N, 5 µg), P und L (pTIT-P und pTIT-L, 2.5 µg pro Plasmid) kodieren, wurden mit der jeweiligen viralen cDNA in ungefähr 10⁶ BSR T7/5 Zellen ko-transfiziert, die die RNA Polymerase des Phagen T7 stabil exprimieren (5). Das Transfektionsmedium wurde nach 4 Stunden abgenommen und durch BHK-21 Medium (Gibco) mit 10% CS ersetzt. Zellkulturüberstände wurden 6 Tage nach Transfektion geerntet und auf neue BSR Zellen gegeben. Der Nachweis infektiöser RV erfolgte durch Immunfärbung mit einem FITC Konjugat (Centocor), das das RV Nucleoprotein N erkennt.

Die Rekombinanten konnten von cDNA in BSR T7/5 Zellen gewonnen werden, die die RV Proteine N, P und L von transfizierten Plasmiden exprimierten. Die Expression der NS Proteine hatte keinen offensichtlichen nachteiligen Effekt auf die Replikation, Wachstumcharakeristika und infektiöse Titer der Rekombinanten in BSR Zellen (nicht gezeigt).

Um die Aktivität der exprimierten BRSV Proteine zu untersuchen, wurden Vero-Zellen mit dem parentalen RV (SAD VB) oder mit jeder einzelnen Rekombinanten infiziert, oder mit beiden Rekombinanten SAD VB-NS1 and SAD VB-NS2 ko-infiziert. Die Infektionen mit den Rekombinanten RV SAD VB, SAD VB-NS1 bzw. SAD VB-NS2 erfolgte wie bereits beschrieben (18) in Suspension, wobei eine MOI von 5 eingesetzt wurde. Für Ko-Infektionen mit SAD VB-NS1 und SAD VB-NS2 wurde pro Rekombinante eine MOI von 2.5 verwendet. Rekombinantes, universelles Typ I Interferon A/D wurde bis zu Konzentrationen von 500 U/ml direkt nach dem Umsetzen der Zellen zugegeben. Die Virustiter wurden zwei Tage nach Infektion durch Verdünnungsreihen und Immunfärbung mit einem gegen das RV Protein N gerichteten FITC Konjugat (Centocor) bestimmt. Zusätzlich wurde die Expression der RV Proteine zwei Tage nach Infektion in mindestens 4 unabhängigen Experimenten überprüft.

Das Wachstum des parentalen RV SAD VB und der NS1- oder NS2 Proteinexprimierenden Viren aus den Einzelinfektionen war gleich stark beeinträchtigt (Fig. 8A). Bei Zugabe von 50 IU IFN alpha nahmen die Titer um ungefähr 1 log ab und fielen dann sehr langsam mit steigenden IFN Mengen weiterhin ab. Dies deutet auf eine schwache IFN Antwort der Vero-Zellen, oder eine hohe intrinsische Resistenz des RV gegenüber der IFN-vermittelten Antwort in Vero-Zellen hin. In Zellen, die mit NS1- und NS2-exprimierenden Viren ko-infiziert waren, konnte jedoch ein Schutz der Virusreplikation gezeigt werden. Die Virustiter blieben signifikant höher als in den Einzelinfektionen und nahmen nur langsam Dosis-abhängig ab.

Um nochmals die obige Beobachtung zu überprüfen, daß die NS Proteine von BRSV die antivirale Antwort boviner Zellen effizienter abwehren können als die in Vero-Zellen, wurden parallel Versuche in MDKB-Zellen durchgeführt (Fig. 8B). Standard RV SAD VB und die NS-exprimierenden Rekombinanten replizierten in unbehandelten MDKB-Zellen zu geringfügig niedrigeren Titern als in Vero-Zellen. Im Gegensatz zu Vero-Zellen reduzierte die IFN Behandlung die infektiösen Titer der Einzelinfektionen von wt RV und der NS-exprimierenden Viren um ein Beträchtliches. Ein unmittelbarer Abfall der infektiösen Titer um 3 log-Stufen wies auf eine höchst wirkungsvolle IFN-vermittelte Zellantwort hin. Trotz dieser Antwort war in Zellen, die mit SAD VB-NS 1 und SAD VB-NS2 ko-infiziert waren, die Virusreplikation jedoch bis zu eingesetzten Mengen von 150 IU IFN vollkommen geschützt. Diese Ergebnisse konnte durch die Analyse der RV Proteinsynthese bestätigt werden. In nichtbehandelten Zellen produzierten alle Rekombinanten vergleichbare Mengen an RV Proteinen, während hingegen in IFN-behandelten Zellen nur die Ko-Infektionen zu einer wesentlichen Proteinsynthese in der Lage waren, bis mehr als 150-200 IU zugegeben wurden (nicht gezeigt).

Die obigen Ergebnisse zeigen, daß die beiden BRSV NS Proteine nicht nur in der Lage sind, dem BRSV Resistenz gegenüber der IFN vermittelten antiviralen Antwort zu verleihen, sondern auch einem anderen, nicht verwandten Virus. Zusätzlich bestätigen die Ergebnisse, daß beide NS Proteine notwendig und ausreichend sind, die IFN-antagonistische Aktivität auszuüben.

### Beispiel 7: Interferon-Resistenz von klinischen Isolaten des humanen RSV (HRSV)

Um zu zeigen, daß auch HRSV Abwehrmechanismen gegenüber Interferon besitzt, wurden Experimente mit klinischen Isolaten von hospitalisierten Patienten durchgeführt. Die Isolate stammen aus einer Multicenter Studie, die von Prof. Werchau an der Ruhr-Universität Bochum koordiniert wurde. Vier Isolate stammen von Patienten, die mit der Diagnose "Bronchiolitis" (#61; #86; #109 und #110; Gruppe 1) und drei Isolate von Patienten, die mit der Diagnose "obstruktive Bronchitis" (#104; #112 und #162; Gruppe 2) in deutschen Krankenhäusern behandelt wurden.

Um eine Adaptierung dieser klinischen Isolate an Zellinien zu verhindern, wurden sie in nur zwei Passagen auf Hep2 Zellen vermehrt und in den nachfolgenden Experimenten eingesetzt. Wachstumskurven dieser Isolate wurden auf Hep2 Zellen bestimmt. Während die Isolate #61; #86 und #109 der Gruppe 1 ebenso wie das Isolat #112 der Gruppe 2 in Hep2 Zellen schnell wuchsen und nach 3 Tagen zu ähnlich hohen infektiösen Titern zwischen 3x10⁶ und 4x10⁷ pfu/ml heranwuchsen, waren die Isolate #110 der Gruppe 1 sowie #104 und #162 der Gruppe 2 im Wachstum deutlich attenuiert und erzielten nach 3 Tagen nur Titer von 3x10⁴ pfu/ml (Abb. 9). Diese Unterschiede im Wachstum konnten auch in primären respiratorischen Epithelzellen beobachtet werden: Während #61, #86, #109 und #112 in deutliche Syncitienbildung induzierten, waren bei den Isolaten #110, #104 und #162 nach der gleichen Infektionsdauer nur Einzelzellinfektionen nachweisbar (nicht gezeigt).

Um die Interferon-Resistenz der einzelnen Isolate zu bestimmen, wurden Hep2-Zellen für 1 Stunde mit den verschiedenen Isolaten infiziert (M.O.I =0,1), danach wurde rekombinantes Typ I Interferon A/D in Konzentrationen von 150 bis 5,000 U/ml appliziert. Nach 72 Stunden Inkubationsdauer wurden die Virustiter der einzelnen klinischen Isolate mittels Endpunkttitration bestimmt. Dabei zeigte sich, daß alle klinischen HRSV-Isolate unabhängig von ihrer Wachstumsgeschwindigkeit vor der antiviralen Wirkung des rekombinanten Typ I IFNs geschützt waren. Erst bei sehr hohen IFN-Konzentrationen von 5,000 U/ml wurde die Replikationsfähigkeit der verschiedenen klinischen HRSV-Isolate um lediglich das 10-fache vermindert (Abb. 10). Diese Daten zeigen, dass alle klinischen HRSV Isolate in der Lage sind, der antiviralen Wirkung hoher Konzentrationen rekombinanten Typ I IFNs entgegenzuwirken, und zwar in mindestens gleichem Maße wie der Laborstamm HRSV Long (vergl. Abb. 7).

Die NS1 und NS2 Gene der Isolate wurde durch RT-PCR amplifiziert und deren Sequenz bestimmt. Dabei zeigte sich, dass beide Gene hoch konserviert sind, was die Ergebnisse der IFN-Assays bestätigt. Sowohl beim NS1 als auch beim NS2 Protein traten gegenüber dem Stamm HRSV Long und unter den Isolaten nur sehr geringe Unterschiede in der Aminosäurensequenz auf. Das NS1 Protein des HRSV Long und die NS1 Proteine der klinischen Isolate # 104, 112, 61 und 110 sind identisch. Bei den Isolaten #162, 86 und 109 besteht jeweils ein Aminosäureaustausch gegenüber der Sequenz des HRSV Long (#86: N(76)S, #162: V(82)M, #109 E(91)G). Die NS2 Proteine aller klinischen Isolate sind in ihrer Aminosäuresequenz identisch und unterscheiden sich von HRSV Long durch 2 Austausche DN(7,8)GT und T(21)I. Für die weiteren Versuche wurden deshalb die NS Proteine bzw. Gene des HRSV Long benutzt.

### Beispiel 8: Die NS1 und NS2 Proteine des humanen RSV (HRSV) verstärkten gemeinsam die Resistenz von Rabies Virus gegenüber der IFN-vermittelten antiviralen Antwort

Um zu zeigen, daß die beiden NS Proteine des HRSV IFN Typ I antagonistische Wirkung haben und diese Funktion auf ein anderes, nichtverwandtes Virus übertragen werden kann, wurden rekombinante Rabies Viren entwickelt, die entweder NS 1 (SAD VB-hNS1) oder NS2 (SAD VB-hNS2) von HRSV (Stamm Long) exprimieren. Rekombinante RV mit hinzugefügtem NS 1 oder NS2 Gen (Abb. 11) wurden auf der Basis der Gesamtlängen-RV cDNA (SAD L16) konstruiert, die eine zusätzliche Transkriptionsstop- und Restartsequenz in der 3' nichtkodierenden Sequenz des G Gens enthält (SAD VB) (32). Das zusätzliche Gen wurde, wie auch für die NS Gene von BRSV beschrieben, zwischen dem G und L Gen des attenuierten Rabies Virus SAD L16 (Abb. 1B) eingefügt. Die cDNAs der beiden HRSV Gene wurden mittels RT-PCR erhalten. Dazu wurde Gesamt-RNA aus HRSV (Long)-infizierten Vero-Zellen isoliert. Für das NS1 Gen wurden folgende Primer benützt: hNS1-NcoI5' (5'-ATT GAC CAT GGG CAG CAA TTC ATT-3'; Erststrangsynthese und PCR) und hNS1-EcoRI5' (5'-ATT GAG AAT TCT TAT GGA TTA AGA TCA AA-3'), für das NS2 Gen die Primer hNS2-NcoI5' (5'-ATT GAC CAT GGA CAC AAC CCA CA-3') und hNS2-EcoRI3' (5'-ATT GAG AAT TCT TAT GGA TTG AGA TCA TA-3'). Diese PCR Fragmente wurden nach Verdau mit den Restriktionsenzymen NotI und EcoRI in ein ebenso geschnittenes TIT-Plasmid kloniert (pTIT-hNS1, pTIT-hNS2). Das NS1 Gen wurde dann aus pTIT-hNS1 mittels PCR mit den Primem hNS1-NotI5' (5'-TAT GAA GCG GCC GCC CCC TCT CTT CTT TCT ACA GAA AAT GGG CAG CAA TTC ATT GAG-3') und hNS1-EcoRI3' vervielfältigt und mit den Restriktionsenzymen NotI und EcoRI verdaut. Nach einer Auffüll-Reaktion mit Klenow Polymerase wurde das Fragment in die einzige SmaI Stelle des pSAD VB unmittelbar stromabwärts des zusätzlichen Transkriptionsstartsignals eingefügt. Daraus ergab sich pSAD VB-hNS1. Das NS2 Gen wurde mit den Primem hNS2-AseI5' (5'-ATA CTT ATT AAT TGG GGC AAA TAA ATC AGT TCC CCA ACC AGC CAT GGA CAC AAC CCA CAA TG-3') und hNS2-Acc65I3' (5'-ATA AAT GGT ACC AAA AGA TAA CAC TGT GTG AAT TAA ATT TTG AAA AGT GCT TAT GGA TTG AGA TCA TAC TTG-3') aus dem pTIT-hNS2 vervielfältigt, mit AseI und Acc65I geschnitten und nach Auffüllen mit Klenow Polymerase entsprechend dem hNS1 Gen kloniert. Daraus resultierte pSAD VB-hNS2 (Abb. 11).

Rekombinantes RV mit entweder dem NS1 oder dem NS2 Gen von HRSV konnte wie bereits beschrieben (19) nach Transfektion (CaPO₄ Protokoll; Mammalian Transfection Kit, Stratagene) von T7 Promotor-kontrollierten Plasmiden der jeweiligen Virus cDNA (10 µg) gewonnen werden. Plasmide, die für das RV Protein N (pTIT-N, 5 µg), P und L (pTIT-P und pTIT-L, 2.5 µg pro Plasmid) kodieren, wurden mit der jeweiligen viralen cDNA in ungefähr 10⁶ BSR T7/5 Zellen ko-transfiziert, die die RNA Polymerase des Phagen T7 stabil exprimieren (5). Das Transfektionsmedium wurde nach 4 Stunden abgenommen und durch BHK-21 Medium (Gibco) mit 10% CS ersetzt. Zellkulturüberstände wurden 6 Tage nach Transfektion geerntet und auf neue BSR Zellen gegeben. Der Nachweis infektiöser RV erfolgte durch Immunfärbung mit einem FITC Konjugat (Centocor), das das RV Nucleoprotein N erkennt. Die Expression der NS Proteine von HRSV hatte keinen offensichtlichen nachteiligen Effekt auf die Replikation, Wachstumcharakeristika und infektiöse Titer der rekombinanten RV in BSR Zellen (nicht gezeigt).

Um die Aktivität der exprimierten HRSV Proteine zu untersuchen, wurden MDKB-Zellen mit dem parentalen RV (SAD VB) oder mit jeder einzelnen Rekombinanten infiziert, oder mit beiden Rekombinanten SAD VB-hNS1 and SAD VB-hNS2 ko-infiziert. Die Infektionen mit den Rekombinanten RV SAD VB, SAD VB-hNS 1 bzw. SAD VB-hNS2 erfolgte wie bereits beschrieben (18) in Suspension, wobei eine MOI von 5 eingesetzt wurde. Für Ko-Infektionen mit SAD VB-hNS1 und SAD VB-hNS2 wurde pro Rekombinante eine MOI von 2.5 verwendet. Rekombinantes, universelles Typ I Interferon A/D wurde in Konzentrationen von 50-500 U/ml direkt nach dem Umsetzen der Zellen zugegeben. Die Virustiter wurden zwei Tage nach Infektion durch Verdünnungsreihen und Immunfärbung mit einem FITC Konjugat (Centocor) gegen das RV Protein N gerichtet bestimmt. Die IFN-Behandlung reduzierte die infektiösen Titer der Einzelinfektionen von wt RV und der hNS-exprimierenden Viren deutlich. Die Zugabe von nur 50 U IU IFN alpha löste einen Abfall der infektiösen Titer um 3 log₁₀₋Stufen aus. In Zellen, die mit SAD VB-hNS1 und SAD VB-hNS2 ko-infiziert waren, war die Virusreplikation jedoch bis zu eingesetzten Mengen von 150 IU IFN deutlich geschützt (Abb. 11).

Diese Ergebnisse zeigen, daß auch die beiden HRSV NS Proteine in der Lage sind, die zelluläre IFN Antwort zu antagonisieren und einem nichtverwandten Virus Resistenz gegenüber der IFN vermittelten antiviralen Antwort zu verleihen. Zusätzlich bestätigen die Ergebnisse, daß beide NS Proteine notwendig und ausreichend sind, die IFN-antagonistische Aktivität auszuüben.

### Beispiel 9: Die NS1 und NS2 Proteine des murinen Pneumovirus (Pneumonia Virus of Mice; PVM) verstärkten gemeinsam die Resistenz von Rabies Virus gegenüber der IFN-vermittelten antiviralen Antwort

Um zu zeigen, daß auch die beiden NS Proteine des PVM IFN Typ I Resistenz vermitteln und diese Funktion auf Rabies Virus übertragen werden kann, wurden rekombinante Rabies Viren entwickelt, die entweder NS1 (SAD VB-mNS1) oder NS2 (SAD VB-mNS2) des PVM exprimieren. Die cDNAs der beiden PVM Gene wurden von Andrew Easton; University of Warwick, U.K., zur Verfügung gestellt (GenBank Acc. No. D10331; Abb. 18). Rekombinante RV mit hinzugefügtem NS1 oder NS2 Gen (Abb. 11) wurden ebenfalls auf der Basis von SAD VB konstruiert (32; siehe Beispiele 6 und 8). Das zusätzliche Gen wurde, wie vorher für die NS Gene von BRSV oder HRSV beschrieben, zwischen dem G und L Gen des RV SAD L16 eingefügt (Fig. 1B). Die Gene für beide NS Proteine wurden mittels PCR vervielfältigt, wobei dem NS 1 Gen direkt vor dem Translations-Stopcodon 27 zusätzliche Nukleotide eingefügt wurden, die eine interne Region des Influenza Hämagglutinin (HA) Proteins kodieren (HA*-tag).* Dem NS2 Gen wurden direkt vor dem Stopcodon 24 Nukleotide eingefügt, die ein sythetisches FLAG-Peptid kodieren (FLAG-*tag*). Für das NS 1 Gen von PVM wurden dazu folgende Primer verwendet: mNS1- NotlEcoRV5' (5'-AAT GAT ATC GCG GCC GCC CCC TCT CTT CTT TCT ACA GAA ATG GGC TGT AAT GTG ATG ATG-3') und mNS1ha-EcoRI/V3' (5'-AAT GAT ATC GAA TTC TTA AGC GTA ATC GG TAC ATC ATA AGG ATA ACC ACT GAT CAG CTC TAC-3'), für das NS2 Gen von PVM die Primer mNS2-AseIEcoRV5' (5'-AAT GAT ATC ATT AAT TGG GGC AAA TAA ATC AGT TCC CCA ACC AGC CAT GTC CAC AGC TAT GAA CAA G-3') und mNS2fl-EcoRI/V3' (5'-AAT GAT ATC GAA TTC TCA TTT ATC GTC ATC ATC TTT ATA GTC ATC ATC ATC CTC ATC-3'). Diese PCR Fragmente wurden dann nach Verdau mit dem Restriktionsenzym EcoRV in die einzige SmaI Stelle des pSAD VB unmittelbar stromabwärts des zusätzlichen Transkriptionsstartsignals eingefügt. Daraus ergaben sich pSAD VB-mNS 1 und pSAD VB-mNS2 (Abb. 11).

Rekombinantes RV mit entweder dem NS1 oder dem NS2 Gen von PVM konnte wie bereits in den Beispielen 6 und 8 beschrieben in BSR T7/5 Zellen gewonnen werden, die die RV Proteine N, P und L von transfizierten Plasmiden exprimierten. Die Expression der NS Proteine hatte keinen offensichtlichen nachteiligen Effekt auf die Replikation, Wachstumcharakeristika und infektiöse Titer der Rekombinanten in BSR Zellen (nicht gezeigt).

Um die Aktivität der exprimierten PVM Proteine zu untersuchen, wurden MDKB-Zellen mit dem parentalen RV (SAD VB) oder mit jeder einzelnen Rekombinanten infiziert, oder mit beiden Rekombinanten SAD VB-mNS1 and SAD VB-mNS2 ko-infiziert. Die Infektionen mit den Rekombinanten RV SAD VB, SAD VB-mNS 1 bzw. SAD VB-mNS2 erfolgte wie bereits beschrieben (18) in Suspension, wobei eine MOI von 5 eingesetzt wurde. Für Ko-Infektionen mit SAD VB-mNS1 und SAD VB-mNS2 wurde pro Rekombinante eine MOI von 2.5 verwendet. Rekombinantes, universelles Typ I Interferon A/D wurde in Konzentrationen von 50-500 U/ml direkt nach dem Umsetzen der Zellen zugegeben. Die Virustiter wurden zwei Tage nach Infektion durch Verdünnungsreihen und Immunfärbung mit einem FITC Konjugat (Centocor) gegen das RV Protein N gerichtet bestimmt. Die IFN Behandlung reduzierte die infektiösen Titer der Einzelinfektionen von wt RV und der hNS-exprimierenden Viren deutlich. Die Zugabe von nur 50 U IU IFN alpha löste einen Abfall der infektiösen Titer um 3 log-Stufen aus. In Zellen, die mit SAD VB-mNS1 und SAD VB-mNS2 ko-infiziert waren, war die Virusreplikation jedoch bis zu eingesetzen Mengen von 100 IU IFN deutlich geschützt (Abb. 13).

Diese Ergebnisse zeigen, daß auch die beiden PVM NS Proteine in der Lage sind, die zelluläre IFN Antwort zu antagonisieren und einem nichtverwandten Virus Resistenz gegenüber der IFN vermittelten antiviralen Antwort zu verleihen. Da die Homologie der PVM NS Proteine zu denen des HRSV lediglich 17% (für NS1) bzw. 20% (für NS2) beträgt, konnte die IFN antagonistische Funktion nicht vorhergesagt werden. Wie bereits für die NS Proteine des BRSV und HRSV beobachtet, sind auch im Falle des PVM beide NS Proteine notwendig und ausreichend, die IFN- antagonistische Aktivität auszuüben.

### Beispiel 10: Herstellung von rekombinanten BRSV mit heterologen NS Genen

Weiterhin haben wir rekombinante, chimäre BRSVs konstruiert, bei denen die eigenen NS Gene durch NS Gene anderer Pneumoviren ersetzt wurden.
Zur Herstellung von rekombinanten BRSVs, die das NS1 Gen von HRSV oder PVM anstelle des BRSV NS1 Gen enthalten, wurde ein Plasmid verwendet, das die nt 1 bis 957 der Gesamtlängen-BRSV cDNA enthält (5) und das zusätzlich im NS1 Gen am nt 311 eine EcoRI Schnittstelle aufweist (pbNS1EcoRIbNS2). Damit kann durch Verdau mit den Restriktionsenzymen NotI und EcoRI die gesamte kodierende Region des NS 1 Gen entfernt werden. Das HRSV und das PVM NS1 Gen wurden mittels PCR unter Verwendung der Primer hNS1-NotI5' und hNS1-EcoRI3' für HRSV NS1 bzw. mNS1-NotIEcoRV5' und mNS1ha-EcoRI3' für PVM NS 1 vervielfältigt und nach Verdau mit den Restriktionsenzymen NotI und EcoRI in das Plasmid eingefügt, wodurch phNS1bNS2 bzw. prnNS1bNS2 entstanden. Beide Plasmide wurden anschließend mit NotI und Acc65I verdaut und die entstehenden Fragmente von 1094 nt Länge für hNS1bNS2 bzw 1043 nt für mNS1bNS2 wurden in die Gesamtlängen-BRSV cDNA gesetzt. Daraus resultierten rBRSV hNS1bNS2 und rBRSV mNS1bNS2 (Fig. 14).

Zur Herstellung von BRSVs mit heterologen NS2 Genen (rBRSV bNS1hNS2 bzw. rBRSV bNS1mNS2) wurde pNSINS2 (5) zuerst mit dem Restriktionsenzym Acc65I und anschließend partiell mit dem Restriktionsenzym AseI verdaut, wodurch ein 446 nt großes Fragment, das das bNS2 Gen beinhaltet, herausgeschnitten wird. An diese Stelle wurde dann ein Fragment gesetzt, das das hNS2 bzw. das mNS2 Gen enthielt. Diese Fragmente wurden mittels PCR generiert unter der Verwendung der Primer hNS2-AseI5' und hNS2-Acc65I3' für das HRSV NS2 Gen bzw. mNS2-AseIEcoRV5' und mNS2-Acc65I3' (5'-ATA AAT GGT ACC AAA AGA TAA CAC TGT GTG AAT TAA ATT TTG AAA AGT GCT CAT TTA TCG TCA TCA TCT TTA TAG-3') für das PVM NS2 Gen. Anschließend wurden die Fragmente mit den Restriktionsenzymen AseI und Acc6I verdaut und in pNSINS2 gesetzt, so daß pbNS1hNS2 bzw. pbNS1mNS2 entstand. Diese Plasmide wurden dann mit den Restriktionsenzymen NotI und Acc65I verdaut und das jeweilige Fragment (949 nt für bNS1hNS2 und 1069 nt für bNS1mNS2) in die Gesamtlängen-BRSV cDNA gesetzt, wodurch rBRSV bNS1hNS2 und rBRSV bNS1mNS2 entstanden (Abb. 14).

Zur Herstellung von rekombinanten BRSVs, die beide NS Gene von HRSV bzw. PVM enthalten wurden die Plasmide phNS 1 bNS2 bzw. pmNS1bNS2 zuerst mit dem Restriktionsenzym Acc65I und dann partiell mit dem Restriktionsenzym AseI verdaut. Anschließend wurden die oben beschriebenen Fragmente für das HRSV NS2 Gen und das PVM NS2 Gen in das jeweilige Plasmid eingesetzt, wodurch phNS1hNS2 bzw. pmNS1mNS2 entstand. Auch diese Plasmide wurden mit den Restriktionsenzymen NotI und Acc65I verdaut und die entstehenden Fragmente von 1094 nt Länge für hNS1hNS2 bzw. 1163 nt Länge für mNS1mNS2 in die Gesamtlängen-cDNA von BRSV gesetzt. Daraus resultierten rBRSV hNS1hNS2 und rBRSV mNS1mNS2 (Abb. 14).

Lebensfähige rekombinante Viren rBRSV hNS1bNS2, rBRSV bNS1hNS2 und rBRSV hNS1hNS2 bzw. rBRSV mNS1bNS2, rBRSV bNS1mNS2 und rBRSV mNS1mNS2 konnten von den jeweiligen cDNA Konstrukten in BSR T7/5 Zellen (5) nach Transfektion (CaPO₄ Protokoll; Mammalian Transfection Kit, Stratagene) von T7 Promotor-kontrollierten Plasmiden mit der jeweiligen Virus cDNA (10 µg) gewonnen werden. Plasmide, die für die BRSV Proteine N und P (pTITB-N und pTITB-P, 4 µg pro Plasmid) und L und M2 (pTITB-L and pTITB-M2, 2 µg pro Plasmid) kodieren, wurden mit der jeweiligen Virus cDNA in ungefähr 10⁶ BSR T7/5 Zellen, die die RNA Polymerase des Phagen T7 stabil exprimieren (5), ko-transfiziert. Nach 4 Stunden wurde das Transfektionsmedium abgenommen und BHK-21 Medium (Gibco) mit 5% FCS zugegeben. Mit BRSV cDNA transfizierte Zellen wurden alle 5 Tage im Verhältnis 1:3 umgesetzt bis ein cytopathogener Effekt sichtbar wurde.
In allen Fällen resultierte die Ko-Transfektion der für die BRSV Proteine N, P, L und M2 kodierenden Support-Plasmide in der Bildung von Syncytien. Die Viren wurden nach Umsetzen der transfizierten Zellen im Verhältnis 1:3 und dem Auftreten eines deutlichen cytopathogenen Effekts geerntet.

Für die Herstellung von Virus-Stammlösungen wurden zu 80% konfluente Vero-Zellen mit einer Multiplizität der Infektion (MOI) von 0.1 in Serum-freiem Dulbecco's minimal essential medium (DMEM) infiziert. Nach einstündiger Adsorption wurde das Inoculum entfernt und die Zellen bei 37°C in DMEM, das mit 2,5% FCS ergänzt war, in einer 5%-igen CO₂-Atmosphäre inkubiert bis nach ca. 4 Tagen ein deutlicher cytopathischer Effekt (CPE) zu sehen war. Die Viren wurden durch Frieren und anschließendes Auftauen freigesetzt. Die Virustiter wurden auf Vero-Zellen durch Verdünnungsreihen in Mikrowell-Platten und anschließendem Auszählen der infizierten Foci bestimmt. Dabei wurden die Foci durch indirekte Färbung mit einem Antikörper gegen das Fusionsprotein F angefärbt (SEROTEC U.K.).

### Beispiel 11: Wachstum der BRSV Chimären: die IFN Resistenz ist Zelltypabhängig

Die Wachstumscharakeristika der Viren wurden zunächst in Vero-Zellen analysiert. Während sich BRSV hNS1hNS2 und BRSV hNS1bNS2 genauso wie BRSV wt verhielt, waren BRSV mNS1mNS2 und BRSV mNS1bNS2 im Vergleich zum parentalen Gesamtlängen-Virus leicht attenuiert. Dies weist darauf hin, daß die Funktion(en) der BRSV NS Proteine bei der Virusreplikation von den HRSV NS Proteinen in vollem Umfang übernommen werden können. Sowohl das parentale wt BRSV als auch die beiden HRSV/BRSV Chimären erreichten infektiöse Titer von ca. 5x10⁵ pfu nach Infektion von Vero-Zellen mit einer MOI von 0.1 und anschließender Inkubationszeit von 3 Tagen (Abb. 15). Im Gegensatz dazu erreichten die PVM/BRSV Chimären nach 3 Tagen nur ca. 4x10⁵ pfu, was darauf hinweist, daß die PVM NS Gene die Funktion der BRSV (und HRSV) Gene bei der Virusreplikation nicht optimal wahrnehmen können.

Anschließend wurde eine Zellinie bovinen Ursprungs, MDBK, verwendet, die das Wachstum des wt BRSV optimal unterstützt und die im Gegensatz zu Vero-Zellen über ein funktionelles IFN Typl System verfügt (5). Hier war das Wachstum von BRSVhNS1hNS2 im Vergleich zum wt BRSV eingeschränkt. Nach 3 Tagen wurden nur Titer von 4x10⁴ pfu erreicht, im Vergleich dazu wächst das wt BRSV bis zu 1x10⁶ pfu (Abb. 16). In der optimalen Wirtszelle für BRSV, den bovinen MDKB-Zellen, ist das Wachstum von BRSV hNS1hNS2 offensichtlich attenuiert, während hingegen in IFNnegativen Vero-Zellen keine Attenuierung festzustellen ist.

Rekombinantes humanes Typ I Interferon wurde eingesetzt, um das Verhalten des wt und BRSV hNS1hNS2 in IFN-stimulierten Zellen zu analysieren. Um den Effekt des Typ I Interferons auf die Replikation der rekombinanten BRSV zu untersuchen, wurden Vero oder MDKB-Zellen wie oben beschrieben mit den verschiedenen Viren mit einer MOI von 0.1 infiziert und in six-well Schälchen in DMEM mit 2.5% FCS ausgesät.

Rekombinantes universelles Typ I Interferon (humanes Interferon alpha A/D, PBL Biomedical Laboratories) wurde in Konzentrationen von 500-10000 U/ml direkt nach dem Aussäen zugegeben. Die Virustiter wurden nach einer dreitägigen Inkubationszeit durch Verdünnungsreihen und indirektem Anfärben der infizierten Zellfoci mit einem Antikörper gegen das Fusionsprotein F bestimmt.

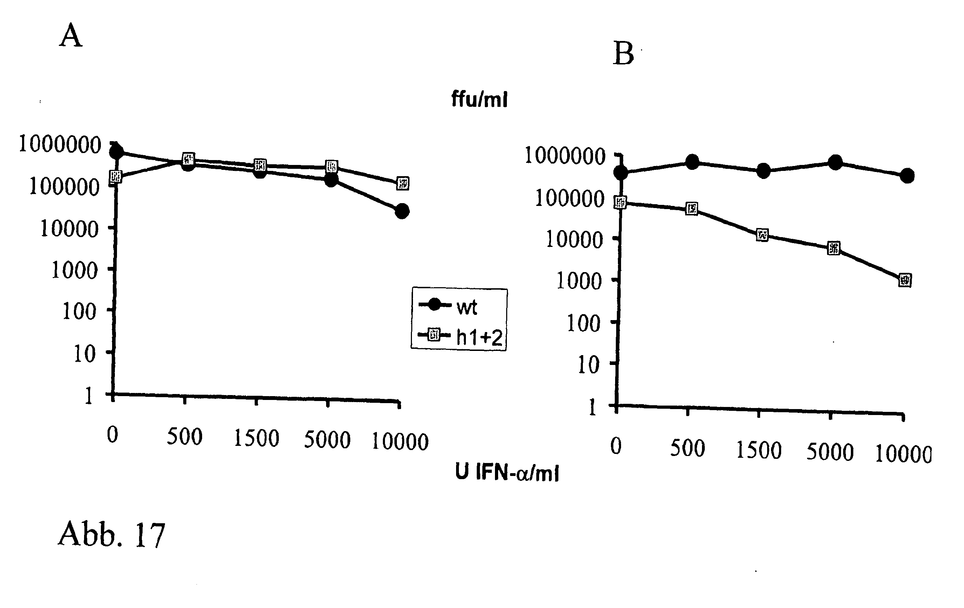

Auf Vero-Zellen konnte kein Unterschied zwischen wt BRSV und BRSV hNS1hNS2 bezüglich ihrer IFN Resistenz festgestellt werden. Bei beiden Viren konnte erst ab 5000 U eine leichte Reduktion der infektiösen Titer festgestellt werden (Abb. 17A). Auf MDKB-Zellen hingegen zeigte BRSV hNS1hNS2 eine starke, dosisabhängige Sensitivität gegenüber der durch IFN induzierten zellulären Antwort, wobei schon 1500 U zu einer 3-fachen, und 10000 U zu einer etwa 30-fachen Reduktion der infektiösen Titer führte. Im Gegensatz dazu zeigte sich das wt BRSV sehr resistent gegenüber der IFN Behandlung. Selbst 10,000 U IFN alpha konnten die Replikation des wt BRSV nicht beeinflußen (Abb. 17B). Obwohl das chimäre BRSV hNS 1 hNS2 auf Vero-Zellen eine ähnliche IFN Resistenz wie das wt BRSV aufweist, kann es die IFN induzierte antivirale Antwort in Zellen bovinen Ursprungs nicht vollständig unterbinden. Dies zeigt, daß die NS Proteine des BRSV die bovine zelluläre antivirale Antwort erfolgreicher bewältigen können als die NS Proteine des HRSV. Offensichtlich ist die IFN-antagonistische Wirkung der NS Proteine in Zellen des heterologen Wirtes suboptimal. Chimäre RS Viren mit heterologen NS Proteinen sind *in vivo* attenuiert und können als Lebendvakzine eingesetzt werden.

### LITERATUR

1. Ahmadian, G., P. Chambers, and A.J. Easton. 1999. Detection and characterization of proteins encoded by the second ORF of the M2 gene of pneumoviruses. J Gen Virol 80 ( Pt 8):2011-2016.
2. Atreya, P.L. and S. Kulkarni. 1999. Respiratory syncytial virus strain A2 is resistant to the antiviral effects of type 1 interferons and human MxA. Virology 261:227-241.
3. Atreya, P.L., M.E. Peeples, and P.L. Collins. 1998. The NS1 protein of human respiratory syncytial virus is a potent inhibitor of minigenome transcription and RNA replication. J Virol 72:1452-1461.
4. Bermingham, A. and P.L. Collins. 1999. The M2-2 protein of human respiratory syncytial virus is a regulatory factor involved in the balance between RNA replication and transcription. Proc Natl Acad Sci U S A 96:11259-11264.
5. Buchholz, U.J., S. Finke, and K.K. Conzelmann. 1999. Generation of bovine respiratory syncytial virus (BRSV) from cDNA: BRSV NS2 is not essential for virus replication in tissue culture, and the human RSV leader region acts as a functional BRSV genome promoter. J Virol 73:251-259.
6. Buchholz, U.J., H. Granzow, K. Schuldt, S.S. Whitehead, B.R. Murphy, and P.L. Collins. 2000. Chimeric bovine respiratory syncytial virus with glycoprotein gene substitutions from human respiratory syncytial virus (HRSV): Effects on host range and evaluation as a live-attenuated HRSV vaccine. J.Virol. 74:1187-1199.
7. Chaplin, P.J., K.R. Parsons, and R.A. Collins. 1996. The cloning of cattle interferon-A subtypes isolated from the gut epithelium of rotavirus-infected calves. Immunogenetics 44:143-145.
8. Collins, P.L., M.G. Hill, E. Camargo, H. Grosfeld, R.M. Chanock, and B.R. Murphy. 1995. Production of infectious human respiratory syncytial virus from cloned cDNA confirms an essential role for the transcription elongation factor from the 5' proximal open reading frame of the M2 mRNA in gene expression and provides a capability for vaccine development. Proc.Natl.Acad.Sci.U.S.A. 92:11563-11567.
9. Collins, P.L., K. McIntosh, and R.M. Chanock. 1996. Respiratory syncytial virus, p. 1313-1352. In B.N. Fields, D.M. Knipe, P.M. Howley, R.M. Chanock, J.L. elnik, T.P. onath, B. oizman, and S.E. traus (eds.), Fields virology. Lippincott-Raven, Philadelphia, PA.
10. Collins, P.L. and G.W. Wertz. 1985. Nucleotide sequences of the 1B and 1C nonstructural protein mRNAs of human respiratory syncytial virus. Virology 143:442-451.
11. Conzelmann, K.K. 1998. Nonsegmented negative-strand RNA viruses: Genetics and manipulation of viral genomes. Annu.Rev.Genet. 32:123-162.
12. Conzelmann, K.K., J.H. Cox, L.G. Schneider, and H.J. Thiel. 1990. Molecular cloning and complete nucleotide sequence of the attenuated rabies virus SAD B19. Virology 175:485-499.
13. Didcock, L., D.F. Young, S. Goodbourn, and R.E. Randall. 1999a. Sendai virus and simian on antitermination protein of respiratory syncytial virus in sequential transcription. J Virol 73:5852-5864.
18. Finke, S. and K.K. Conzelmann. 1997. Ambisense gene expression from recombinant rabies virus: random packaging of positive- and negative-strand ribonucleoprotein complexes into rabies virions. J.Virol. 71:7281-7288.
19. Finke, S. and K.K. Conzelmann. 1999. Virus promoters determine interference by defective RNAs: selective amplification of mini-RNA vectors and rescue from cDNA by a 3' copy-back ambisense rabies virus. J Virol 73:3818-3825.
20. Gale, M.J., C.M. Blakely, B. Kwieciszewski, S.L. Tan, M. Dossett, N.M. Tang, M.J. Korth, S.J. Polyak, D.R. Gretch, and M.G. Katze. 1998. Control of PKR protein kinase by hepatitis C virus nonstructural 5A protein: molecular mechanisms of kinase regulation. Mol Cell Biol 18:5208-5218.
21. Gale, M.J.J., M.J. Korth, N.M. Tang, S.L. Tan, D.A. Hopkins, T.E. Dever, S.J. Polyak, D.R. Gretch, and M.G. Katze . 1997. Evidence that hepatitis C virus resistance to interferon is mediated through repression of the PKR protein kinase by the nonstructural 5A protein. Virology 230:217-227.
22. Garcin, D., P. Latorre, and D. Kolakofsky. 1999. Sendai virus C proteins counteract the interferon-mediated induction of an antiviral state. J Virol 73:6559-6565.
23. Garcia-Sastre, A., R.K. Durbin, H. Zheng, P. Palese, R. Gertner, D.E. Levy, and J.E. Durbin. 1998. The rote of interferon in influenza virus tissue tropism. J Virol 72:8550-8558.
24. Garcia-Sastre, A., A. Egorov, D. Matassov, S. Brandt, D.E. Levy, J.E. Durbin, P. Palese, and T. Muster. 1998. Influenza A virus lacking the NS1 gene replicates in interferon-deficient systems. Virology 252:324-330.
25. Grosteld, H., M.G. Hill, and P.L. Collins. 1995. RNA replication by respiratory syncytial virus (RSV) is directed by the N, P, and L proteins; transcription also occurs under these conditions but requires RSV superinfection for efficient synthesis of full-length mRNA. J.Virol. 69:5677-5686.
26. Hanada, N., T. Morishima, K. Nishikawa, S. Isomura, and Y. Nagai. 1986. Interferon-mediated self-limiting growth of respiratory syncytial virus in mouse embryo cells. J Med Virol 20:363-370.
27. Hardy, R.W. and G.W. Wertz. 1998. The product of the respiratory syncytial virus M2 gene ORF1 enhances readthrough of intergenic junctions during viral transcription. J Virol 72:520-526.
28. Jin, H., X. Cheng, H.Z. Zhou, S. Li, and R. Seid. 2000. Respiratory syncytial virus that lacks open reading frame 2 of the M2 gene (M2-2) has altered growth characteristics and is attenuated in rodents. J.Virol. 74:74-82.
29. Jin, H., D. Clarke, H.Z. Zhou, X. Cheng, K. Coelingh, M. Bryant, and S. Li. 1998. Recombinant human respiratory syncytial virus (RSV) from cDNA and construction of subgroup A and B chimeric RSV. Virology 251:206-214.
30. Lerch, R.A., E.J. Stott, and G.W. Wertz. 1989. Characterization of bovine respiratory syncytial virus proteins and mRNAs and generation of cDNA clones to the viral mRNAs. J Virol 63:833-840.
31. Mallipeddi, S.K., S.K. Samal, and S.B. Mohanty. 1990. Analysis of polypeptides synthesized in bovine respiratory syncytial virus-infected cells. Arch Virol 115:23-36.
32. Mebatsion, T., M.J. Schnell, J.H. Cox, S. Finke, and K.K. Conzelmann. 1996. Highly stable expression of a foreign gene from rabies virus vectors. Proc.Natl.Acad.Sci.U.S.A. 93:7310-7314.
33. Mohanty, S.B., A.L. Ingling, and M.G. Lillie. 1975. Experimentally induced respiratory syncytial viral infection in calves. Am J Vet Res 36:417-419.
34. Pastey, M.K. and S.K. Samal. 1995. Nucleotide sequence analysis of the nonstructural NS1 (1C) and NS2 (1B) protein genes of bovine respiratory syncytial virus. J.Gen.Virol. 76:193-197.
35. Ploegh, H.L. 1998. Viral strategies of immune evasion. Science 280:248-253.
36. Pringle, C.R. 1996. Virus taxonomy 1996 - a bulletin from the Xth International Congress of Virology in Jerusalem. Arch Virol 141:2251-2256.
37. Pringle, C.R. 1998. The universal system of virus taxonomy of the International Committee on Virus Taxonomy (ICTV), including new proposals ratified since publication of the Sixth ICTV Report in 1995 [news] [published erratum appears in Arch Virol 1998;143(3):630]. Arch Virol 143:203-210.
38. Samal, S.K., M.K. Pastey, T.H. McPhillips, and S.B. Mohanty. 1993. Bovine respiratory syncytial virus nucleocapsid protein expressed in insect cells specifically interacts with the phosphoprotein and the M2 protein. Virology 193:470-473.
39. Schnell, M.J., T. Mebatsion, and K.K. Conzelmann. 1994. Infectious rabies viruses from cloned cDNA. EMBO J. 13:4195-4203.
40. Stott, E.J., L.H. Thomas, G. Taylor, A.P. Collins, J. Jebbett, and S. Crouch. 1984. A comparison of three vaccines against respiratory syncytial virus in calves. J Hyg (Lond) 93:251-261.
41. Tan, S.L. and M.G. Katze. 1998. Biochemical and genetic evidence for complex formation between the influenza A virus NS1 protein and the interferon- induced PKR protein kinase. J Interferon Cytokine Res 18:757-766.
42. Taylor, D.R., S.T. Shi, P.R. Romano, G.N. Barber, and M.M. Lai. 1999. Inhibition of the interferon-inducible protein kinase PKR by HCV E2 protein [see comments]. Science 285:107-110.
43. Teng, M.N. and P.L. Collins. 1999. Altered growth characteristics of recombinant respiratory syncytial viruses which do not produce NS2 protein. J Virol 73:466-473.
44. Uze, G., G. Lutfalla, and K.E. Mogensen. 1995. Alpha and beta interferons and their receptor and their friends and relations. J Interferon Cytokine Res 15:3-26.
45. van der Poel, W.H., A. Brand, J.A. Kramps, and J.T. van Oirschot. 1994. Respiratory syncytial virus infections in human beings and in cattle. J Infect 29:215-228.
46. Wathelet, M.G., P.M. Berr, and G.A. Huez. 1992. Regulation of gene expression by cytokines and virus in human cells lacking the type-I interferon locus. Eur J Biochem 206:901-910.
47. Weber, E., B. Humbert, H.J. Streckert, and H. Werchau. 1995. Nonstructural protein 2 (NS2) of respiratory syncytial virus (RSV) detected by an antipeptide serum. Respiration 62:27-33.
48. Whitehead, S.S., A. Bukreyev, M.N. Teng, C.Y. Firestone, M. St Claire, W.R. Elkins, P.L. Collins, and B.R. Murphy. 1999. Recombinant respiratory syncytial virus bearing a deletion of either the NS2 or SH gene is attenuated in chimpanzees. J Virol 73:3438-3442.
49. Yu, Q., R.W. Hardy, and G.W. Wertz. 1995. Functional cDNA clones of the human respiratory syncytial (RS) virus N, P, and L proteins support replication of RS virus genomic RNA analogs and define minimal trans-acting requirements for RNA replication. J Virol 69:2412-2419.

## Patentansprüche

1. Die Verwendung eines NS2 Proteins oder einer NS2-kodierenden Nukleinsäuresequenz zur Herstellung einer pharmazeutischen Formulierung zur Reduzierung der IFN-vermittelten Immunantwort.

2. Die Verwendung gemäß Anspruch 1, wobei das RSV NS2 Protein und zusätzlich ein NS 1 Protein oder die NS2 kodierende Nukleinesäuresequenz und zusätzlich eine NS1 kodierende Nukleinsäuresequenz verwendet werden..

3. Die Verwendung gemäß Anspruch 1 oder 2, wobei die Immunantwort eine antivirale Antwort ist.

4. Die Verwendung gemäß den Ansprüchen 1 - 3, wobei die IFN-vermittelte Immunantwort gehemmt wird.

5. Die Verwendung gemäß einem den Ansprüchen 1-4, wobei die Nukleinsäuresequenz DNA oder RNA ist.

6. Die Verwendung gemäß der Ansprüche 2-5, wobei die RSV NS1 und NS2 Proteine zusammen .verwendet werden, oder wobei die NS1-kodierende Nukieinsäuresequenz zusammen mit der NS2-kodierenden Nukleinsäusequenz verwendet wird.

7. Die, Verwendung gemäß Anspruch 6, wobei das RSV NS Protein und das NS2 Protein von verschiedenen RSV Viren abgeleitet sind, oder wobei die RSV NS1-kodierende Nukleinsäuresequenz und die RSV NS2-kodierende Nukleinsauresequenz von verschiedenen RSV Viren abgeleitet sind.

8. Die Verwendung gemäß Anspruch 5, wobei die Nukleinsäuresequenz in einem Plasmid oder in einem Vektor oder in einem Virus-abgeleiteten Vektor oder in einem rekombinanten DNA oder RNA Virus enthalten ist.

9. Die Verwendung gemäß Anspruch 8, wobei der Vektor ein viraler Gen-Expressionsvektor ist der für Immunisierungszwecke, für Gentherapie, oder für zytoreduktive (Zell-Abtötung; Krebs) Therapie verwendet wird.

10. Die Verwendung gemäß Anspruch 8, wobei das Virus oder der Virus-abgeleitete Vektor von einem DNA Virus, wie zB. Adenovirus (AdV), Adeno-assoziiertes Virus (AAV), Herpesvirus, oder Poxvirus abgeleitet ist.

11. Die Verwendung gemäß Anspruch 8, wobei das Virus oder der Virus-abgeleitete Vektor von einem Positiv-Strang Virus abgeleitet ist, vorzugsweise von Viren aus den Familien Alpha-, Flavi-, and Picornaviridae.

12. Die Verwendung gemäß Anspruch 8, wobei das Virus oder der Virus-abgeleitete Vektor von einem segmentierten oder nicht-segmentierten Negativ-Strang RNA Virus abgeleitet ist.

13. Die Verwendung gemäß Anspruch 8, wobei das RNA Virus aus den Familien Paramyxoviridae, Filoviridae, Bomaviridae, oder Rhabdovihdae abgeleitet ist, insbesondere vom Tollwutvirus.

14. Die Verwendung gemäß Anspruch 13, wobei das RNA Virus von humanem oder von Tier-RSV abgeleitet ist.

15. Die, Verwendung gemäß Anspruch 14, wobei die NS2-kodierende Nukleinsäuresequenz heterolog zu dem besagten humanen oder Tier-RSV ist.

16. Die Verwendung gemäß den Amprilchen 1 bis 15, wobei die pharmazeutische Formulierung weiterhin einen Impfstoff umfaßt.

17. Die Verwendung gemäß den Ansprüchen 1 bis 16, wobei das NS2 Protein verändert ist.

18. Die Verwendung gemäß Anspruch 17, wobei die Veränderung aus der Gruppe der Aminosäure-Austausche, -Deletionen, oder -Insertionen ausgewählt wird.

19. Die Verwendung gemäß den Ansprüchen 17 oder 18, wobei die Veränderung in einer Erhöhung der IFN antagonistischen Aktivität des NS2 Proteins resultiert.

20. Die Verwendung gemäß den Ansprüchen 17 oder 18, wobei die Veränderung in einer Reduzierung der IFN antagonistischen Aktivität des NS2 Proteins resultiert.

21. Die Verwendung gemäß der Anspruche 1 bis 20, wobei IFN Typ I Interferon (IFN alpha oder IFN beta) ist.

22. Die Verwendung gemäß Anspruch 1, wobei das NS2 Protein oder die NS2-kodierende Nukleinsäuresequenz von BRSV, HRSV oder PVM stammt.

23. Die Verwendung gemäß Anspruch 2, wobei das RSV NS1 und/oder NS2 Protein oder die NS 1-kodierende Nukleinsäuresequenz und/oder die NS2-kodierende Nukleinsäuresequenz von BRSV, HRSV oder PVM stammt.

24. Die Verwendung gemäß Anspruch 25, wobei das NS2 Protein von BRSV, HRSV oder PVM die Sequenz gemäß Abb. 18 besitzt oder wobei die NS2-kodierende Nukleinsäure von BRSV, HRSV oder PVM für eine Sequenz gemäß Abb. 18 kodiert.

25. Die Verwendung gemäß Anspruch 27, wobei das RSV NS1 und/oder NS2 Protein von BRSV, HRSV oder PVM die Sequenz gemäß Abb. 18 besitzt oder wobei die NS1-kodierende Nukleinsäure und/oder die NS2-kodierende Nukleinsäure von BRSV, HRSV oder PVM für eine Sequenz gemäß Abb. 18 kodiert.

## Claims

1. The use of an RSV NS2 protein or of a nucleic acid sequence encoding NS2 for the preparation of a pharmaceutical formulation for reduction of the IFN mediated immune response.

2. The use according to claim 1 wherein the RSV NS2 and additionally an NS1 protein or the nucleic acid sequence encoding NS2 and additionally a nucleic acid encoding NS1 are used.

3. The use according to claims 1 or 2 wherein the immune response is an antiviral response.

4. The use according to claims 1-3 wherein the IFN mediated immune response is inhibited.

5. The use according to any of the claims 1-4 wherein the nucleic acid sequence is DNA or RNA.

6. The use according to claims 2-5 wherein the RSV NS1 and NS2 proteins are used together or wherein the nucleic acid sequence encoding NS1 is used together with the nucleic acid sequence encoding NS2.

7. The use according to claim 6 wherein the RSV NS1 protein and NS2 protein are derived from different RSV viruses or wherein the nucleic acid sequence encoding RSV NS1 and the nucleic acid sequence encoding RSV NS2 are derived from different RSV viruses.

8. The use according to claim 5 wherein the nucleic acid sequence is contained in a plasmid or in a vector or in a virus derived vector or in a recombinant DNA or RNA virus.

9. The use according to claim 8 wherein said vector is a viral gene expression vector used for immunization purposes, in gene therapy or in cytoreductive (cell destruction; anticancer) therapy.

10. The use according to claim 8 wherein the virus or the virus derived vector is derived from a DNA virus such as adenovirus (AdV), adeno-associated virus (AAV), herpes virus, or pox virus.

11. The use according to claim 8 wherein the virus or the virus derived vector is derived from a positive strand virus, preferably from viruses of the families of Alpha-, Flavi- and Picornaviridae.

12. The use according to claim 8 wherein the virus or the virus derived vector is derived from a segmented or non-segmented negative strand RNA virus.

13. The use according to claim 8 wherein the RNA virus is derived from the families of Paramyxoviridae, Filoviridae, Bornaviridae, or Rhabdoviridae, particularly for rabies virus.

14. The use according to claim 13 wherein the RNA virus is derived from human or animal RSV.

15. The use according to claim 14 wherein the nucleic acid sequence encoding NS2 are heterologous to said human or animal RSV.

16. The use according to claims 1 to 15 wherein the pharmaceutical formulation further contains a vaccine.

17. The use according to claims 1 to 16 wherein the NS2 protein is modified.

18. The use according to claim 17 wherein the modification is selected from the group amino acid substitutions, deletions, or insertions.

19. The use according to claims 17 or 18 wherein the modification results in an increase of the IFN antagonizing activity of the NS2 protein.

20. The use according to claims 17 or 18 wherein the modification results in a reduction of the IFN antagonizing activity of the NS2 protein.

21. The use according to claims 1 to 20 wherein IFN is type I interferon (IFN alpha or IFN beta).

22. The use according to claim 1 the NS2 protein or the nucleic acid sequence encoding NS2 being derived from BRSV, HRSV, or PVM.

23. The use according to claim 2 the RSV NS1 and/or NS2 protein or the nucleic acid sequence encoding NS1 and/or the nucleic acid sequence encoding NS2 being derived from BRSV, HRSV, or PVM.

24. The use according to claim 22 the NS2 protein of BRSV, HRSV, or PVM having the sequence according to Fig. 18 or the nucleic acid sequence encoding NS2 of BRSV, HRSV, or PVM encoding the sequence according to Fig. 18.

25. The use according to claim 23 the RSV NS1 and/or NS2 protein of BRSV, HRSV, or PVM having the sequence according to Fig. 18 or the nucleic acid sequence encoding NS1 and/or the nucleic acid sequence encoding NS2 of BRSV, HRSV, or PVM encoding the sequence according to Fig. 18.

## Revendications

1. Utilisation d'une protéine NS2 de RSV ou d'une séquence d'acide nucléique codant NS2 pour la préparation d'une formulation pharmaceutique destinée à la réduction de la réponse immunitaire médiée par les IFN.

2. Utilisation selon la revendication 1, dans laquelle la NS2 de RSV et en outre une protéine NS1, ou la séquence d'acide nucléique codant NS2 et en outre un acide nucléique codant NS1 sont utilisés.

3. Utilisation selon la revendication 1 ou 2, dans laquelle la réponse immunitaire est une réponse antivirale

4. Utilisation selon les revendications 1 à 3 dans laquelle la réponse immunitaire médiée par les IFN est inhibée.

5. Utilisation selon l'une quelconque des revendications 1 à 4, dans laquelle la séquence d'acide nucléique est un ADN ou un ARN.

6. Utilisation selon les revendications 2 à 5, dans laquelle les protéines NS1 et NS2 de RSV sont utilisées ensemble ou dans laquelle la séquence d'acide nucléique codant NS1 est utilisée avec la séquence d'acide nucléique codant NS2.

7. Utilisation selon la revendication 6, dans laquelle la protéine NS1 et la protéine NS2 de RSV sont dérivées de virus RSV différents ou dans laquelle la séquence d'acide nucléique codant NS1 de RSV et la séquence d'acide nucléique codant NS2 de RSV sont dérivées de virus RSV différents.

8. Utilisation selon la revendication 5, dans laquelle la séquence d'acide nucléique est contenue dans un plasmide ou dans un vecteur ou dans un vecteur dérivé d'un virus ou dans un virus à ADN ou à ARN recombinant.

9. Utilisation selon la revendication 8, dans laquelle ledit vecteur est un vecteur d'expression de gène viral utilisé dans des objectifs d'immunisation, en thérapie génique ou en thérapie cytoréductrice (destruction de cellules ; thérapie anti-cancéreuse).

10. Utilisation selon la revendication 8, dans laquelle le virus ou le vecteur dérivé du virus est dérivé d'un virus à ADN tel qu'un adénovirus (AdV), un virus adéno-associé (AAV), un herpesvirus, ou un poxvirus.

11. Utilisation selon la revendication 8, dans laquelle le virus ou le vecteur dérivé d'un virus est dérivé d'un virus à brin positif, de préférence d'un virus des familles d'Alphaviridae, Flaviviridae et Picornaviridae.

12. Utilisation selon la revendication 8, dans laquelle le virus ou le vecteur dérivé d'un virus est dérivé d'un virus à ARN à brin négatif segmenté ou non segmenté.

13. Utilisation selon la revendication 8, dans laquelle le virus à ARN est dérivé des familles de Paramyxoviridae, Filoviridae, Bornaviridae ou Rhabdoviridae, en particulier de virus de la rage.

14. Utilisation selon la revendication 13, dans laquelle le virus à ARN est dérivé d'un RSV humain ou animal.

15. Utilisation selon la revendication 14, dans laquelle la séquence d'acide nucléique codant NS2 est hétérologue par rapport audit RSV humain ou animal.

16. Utilisation selon les revendications 1 à 15, dans laquelle la formulation pharmaceutique contient en outre un vaccin.

17. Utilisation selon les revendications 1 à 16, dans laquelle la protéine NS2 est modifiée.

18. Utilisation selon la revendication 17, dans laquelle la modification est choisie dans l'ensemble des substitutions, délétions ou insertions d'acides aminés.

19. Utilisation selon la revendication 17 ou 18, dans laquelle la modification provoque une augmentation de l'activité d'antagonisme vis-à-vis d'un IFN de la protéine NS2.

20. Utilisation selon la revendication 17 ou 18, dans laquelle la modification provoque une réduction de l'activité d'antagonisme vis-à-vis d'un IFN de la protéine NS2.

21. Utilisation selon les revendications 1 à 20, dans laquelle l'IFN est un interféron type I (IFN alpha ou IFN bêta).

22. Utilisation selon la revendication 1, la protéine NS2 ou la séquence d'acide nucléique codant NS2 étant dérivée de BSRV, HRSV, ou PVM.

23. Utilisation selon la revendication 2, la protéine NS1 et/ou NS2 de RSV ou la séquence d'acide nucléique codant NS1 et/ou la séquence d'acide nucléique codant NS2 étant dérivées de BRSV, HRSV ou PVM.

24. Utilisation selon la revendication 22, la protéine NS2 de BRSV, HRSV ou PVM ayant la séquence conforme à la figure 18 ou la séquence d'acide nucléique codant NS2 de BRSV, HRSV ou PVM codant la séquence conforme à la figure 18.

25. Utilisation selon la revendication 23, la protéine NS1 et/ou NS2 de BRSV, HRSV ou PVM ayant la séquence conforme à la figure 18 ou la séquence d'acide nucléique codant NS1 et/ou la séquence d'acide nucléique codant NS2 de BRSV, HRSV ou PVM codant la séquence conforme à la figure 18.


### SEQUENZPROTOKOLL

<110> Wyeth
<120> PNEUMOVIRUS NS PROTEINE ANTAGONISIEREN DIE INTERFERON
   (INF) ANTWORT
<130> 128-001 PEP
<140> 01 929 607.8
   <141> 2001-04-26
<150> DE 100 20 505.4
   <151> 2000-04-26
<160> 16
<170> PatentIn Ver. 2.1
<210> 1
   <211> 33
   <212> DNA
   <213> Künstliche Sequenz
<220>
   <223> Beschreibung der künstlichen Sequenz:Primer
   NNot(+)
<400> 1
   taggcggccg caaaaatggc tcttaggaag gtg 33
<210> 2,
   <211> 21
   <212> DNA
   <213> Künstliche Sequenz
<220>
   <223> Beschreibung der künstlichen Sequenz:Primer
   Nstu(-)
<400> 2
   tcctttgtat cgtttcattt c 21
<210> 3
   <211> 60
   <212> DNA
   <213> Künstliche Sequenz
<220>
   <223> Beschreibung der künstlichen Sequenz:Primer
   NS1HAr-EcoRI
<400> 3
   gcaatagaat tcctaagcgt aatctggtac atcataagga taattcagac caagaagagt 60
<210> 4
   <211> 60
   <212> DNA
   <213> Künstliche Sequenz
<220>
   <223> Beschreibung der künstlichen Sequenz: Primer
   NS2FLr-EcoRI
<400> 4
   gcaatagaat tcctatttat cgtcatcatc tttataatct ggatttaaat catacttata 60
<210> 5
   <211> 24
   <212> DNA
   <213> Künstliche Sequenz
<220>
   <223> Beschreibung der künstlichen Sequenz:Primer
   hNS1-NcoI5
<400> 5
   attgaccatg ggcagcaatt catt 24
<210> 6
   <211> 29
   <212> DNA
   <213> Künstliche Sequenz
<220>
   <223> Beschreibung der künstlichen Sequenz:Primer hNS1-EcoRI5
<400> 6
   attgagaatt cttatggatt aagatcaaa 29
<210> 7
   <211> 23
   <212> DNA
   <213> Künstliche Sequenz
<220>
   <223> Beschreibung der künstlichen Sequenz:Primer hNS2-NcoI5
<400> 7
   attgaccatg gacacaaccc aca 23
<210> 8
   <211> 29
   <212> DNA
   <213> Künstliche Sequenz
<220>
   <223> Beschreibung der künstlichen Sequenz:Primer hNS2-EcoRI3
<400> 8
   attgagaatt cttatggatt gagatcata 29
<210> 9
   <211> 57
   <212> DNA
   <213> Künstliche Sequenz
<220>
   <223> Beschreibung der künstlichen Sequenz:Primer hNS1-NotI5
<400> 9
   tatgaagcgg ccgccccctc tcttctttct acagaaaatg ggcagcaatt cattgag 57
<210> 10
   <211> 62
   <212> DNA
   <213> Künstliche Sequenz
<220>
   <223> Beschreibung der künstlichen Sequenz:Primer hNS2-AseI5
<400> 10
   atacttatta attggggcaa ataaatcagt tccccaacca gccatggaca caacccacaa 60
   tg 62
<210> 11
   <211> 71
   <212> DNA
   <213> Künstliche Sequenz
<220>
   <223> Beschreibung der künstlichen Sequenz:Primer hNS2-Acc65I3
<400> 11
   ataaatggta ccaaaagata acactgtgtg aattaaattt gaaaagtgct tatggattga 60
   gatcatactt g   71
<210> 12
   <211> 60
   <212> DNA
   <213> Künstliche Sequenz
<220>
   <223> Beschreibung der künstlichen Sequenz:Primer mNS1-NotIEcoRV5
<400> 12
   aatgatatcg cggccgcccc ctctcttctt tctacagaaa tgggctgtaa tgtgatgatg 60
<210> 13
   <211> 62
   <212> DNA
   <213> Künstliche Sequenz
<220>
   <223> Beschreibung der künstlichen
   Sequenz:mNS1ha-EcoRI/V3
<400> 13
   aatgatatcg aattcttaag cgtaatcggt acatcataag gataaccact gatcagctct 60
   ac 62
<210> 14
   <211> 67
   <212> DNA
   <213> Künstliche Sequenz
<220>
   <223> Beschreibung der künstlichen Sequenz:Primer
   mNS2-AselEcoRV5
<400> 14
   aatgatatca ttaattgggg caaataaatc agttccccaa ccagccatgt ccacagctat 60
   gaacaag                                                           67
<210> 15
   <211> 57
   <212> DNA
   <213> Künstliche Sequenz
<220>
   <223> Beschreibung der künstlichen Sequenz:Primer
   mNS2f1-EcoRI/V3
<400> 15
   aatgatatcg aattctcatt tatcgtcatc atctttatag tcatcatcat cctcatc 57
<210> 16
   <211> 75
   <212> DNA
   <213> Künstliche Sequenz
<220>
   <223> Beschreibung der künstlichen Sequenz:Primer mNS2-Acc65I3
<400> 16
   ataaatggta ccaaaagata acactgtgtg aattaaattt tgaaaagtgc tcatttatcg 60
   tcatcatctt tatag                                                  75